# EUROPEAN PATENT SPECIFICATION

(11) **EP 0 466 730 B1**
(45) Date of publication and mention of the grant of the patent: **21.07.1993**
(21) Application number: 90905160.9
(22) Date of filing: 08.03.1990
(51) Int. Cl.: C07F 9/653, C07F 9/6503, A61K 31/675

(54) **GEMINAL BISPHOSPHONIC ACIDS AND DERIVATIVES AS ANTI-ARTHRITIC AGENTS**
GEMINALE BISPHOSPHONSÄUREN UND DEREN DERIVATE ALS ANTIARTHRITISCHE MITTEL
ACIDES ET DERIVES BIPHOSPHONIQUES GERMINAUX A TITRE D'AGENTS ANTI-ARTHRITIQUES

(30) Priority: 03.04.1989 US 332618
(43) Date of publication of application: 22.01.1992
(73) Proprietor: THE UPJOHN COMPANY, Kalamazoo, Michigan 49001 (US)
(72) Inventor: DUNN, Colin, John, Richland, MI 49083 (US); NUGENT, Richard, Allen, Galesburg, MI 49053 (US)
(74) Representative: Perry, Robert Edward
(86) International application number: US9001106
(87) International publication number: WO9012017

(56) References cited:
- EP-A- 0 282 320
- EP-A- 0 304 962
- DE-A- 3 719 513

## Description

### FIELD OF THE INVENTION

The invention relates to geminal phosphonic acids, esters and salts which are useful as anti-arthritic agents.

### DESCRIPTION OF THE RELATED ART

Russian Chemical Reviews 47, 803 (1978), discloses 1,3-dipolar cycloaddition to unsaturated organophosphorus compounds, to form five member heterocycles which contain a phosphorus atom in a side-chain, such as phosphinyl-Δ²-pyrazolines, 5-phosphinyl-2-isoxazolines and isoxazolidines.

US-A-4746654 discloses bisphosphonates useful as anti-inflammatory agents.

AU-A-51534/85 discloses bisphosphonates useful in treating abnormal calcium and phosphorus metabolism and in treating arthritis.

US-A-3683080 discloses polyphosphonates, in particular diphosphonates, useful in inhibiting anomalous deposition and mobilisation of calcium phosphate in animal tissue.

DE-A-3719513 discloses diphosphonic acid derivatives useful in treatment of disorders of calcium metabolism.

EP-A-0282320 describes isoxazolyl-aminomethylene-bisphosphonic acid derivatives and the anti-arthritic and bone resorption-inhibiting activity of these compounds.

### SUMMARY OF INVENTION

Novel compounds according to this invention are unsaturated geminal phosphonates of formula (III) where
X₁ is -O-, -NH- or -N-metal where metal is sodium, potassium, calcium, magnesium, copper, zinc, barium, silver or gold;
R₁ is H, C₁-C₄ alkyl, C₃-C₇ cycloalkyl, -φ optionally substituted with 1 through 5, -F, -Cl, -Br, -I, -CF₃, C₁-C₆ alkyl, C₁-C₆ cycloalkyl, C₁-C₄ alkoxy or C₁-C₄ alkylthio;
R₂₋₁ is
C₁-C₆ alkyl,
C₃-C₇ cycloalkyl,
-φ optionally substituted with 1 through 5 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
-CH(OH)-R₂₋₅ where R₂₋₅ is
(A) C₁-C₁₀ alkyl,
(B) C₃-C₇ cycloalkyl,
(C) -φ optionally substituted with 1 or 2 -φ or 1 through 5 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(D) 2- and 3-furanyl optionally substituted with 1 through 3 -F, -Cl, -Br, -I, -NO₂, -CN, -CF₃, C₁-C₆ alkyl, C₃-C₇ cycloalkyl, C₁-C₄ alkoxy, -O-φ, C₁-C₄ alkylthio,
(E) 2-, 4- and 5-pyrimidyl optionally substituted with 1 through 3 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(F) 2-, 3- and 4-pyridinyl optionally substituted with 1 through 4 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(G) 2- and 3-thiophene optionally substituted with 1 through 3 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(H) 1- and 2-naphthalene optionally substituted with 1 through 7 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(I) 2-, 3-, 4-, 6-, 7- and 8-quinoline,
(J) 1-, 3-, 4-, 6-, 7- and 8-isoquinoline,
(K) 2-, 3-, 4-, 5-, 6- and 7-benzothiophene,
(L) 2-, 3-, 4-, 5-, 6- and 7-benzofuran,
(M) -NR₂₋₆R₂₋₇ where R₂₋₆ and R₂₋₇ are the same or different and are C₁-C₄ alkyl,
   -φ,
   -CO-R₂₋₈ wherein R₂₋₈ is C₁-C₄ alkyl or -φ optionally substituted with 1 -CH₃,
   -SO₂-R₂₋₈ where R₂₋₈ is as defined above and where R₂₋₆ and R₂₋₇ are taken together with the attached nitrogen atom to form a 4 through 8 member heterocyclic ring containing a nitrogen, oxygen or sulfur heteroatom and 0 thru 3 double bonds,
-CO-R₂₋₅ where R₂₋₅ is as defined above;
R₃ is -H, C₁-C₆ alkyl, -φ and pharmaceutically acceptable salts thereof.

### DETAILED DESCRIPTION OF THE INVENTION

The nitrogen containing dipoles (I) are either known to those skilled in the art or can readily be prepared from known compounds by known methods. For a review of the synthesis of dipoles (I) see, 1,3 Dipolar Cycloaddition Chemistry, edited by Albert Padwa, NY, Wiley, 1984. It is preferred that X₁ is -NH-. With regard to R₂$-$1 it is preferred that _{R2-1} is -CO-R₂₋₅ and -CH(OH)-R₂₋₅. It is more preferred that _{R2-1} is -CO-φ optionally substituted with -F, -Cl, -Br, -I, -CN, -CF₃, C₁-C₄ alkoxy, C₁-C₄4 alkylthio, C₁-C₄ alkyl, -N(CH₃)₂, -N(CH₂CH₃)₂, morpholino, 1-, and 2-naphthalene, 2-thienyl, cyclopropyl and 2-, 3- and 4-pyridyl.

There are two preferred methods of preparing the nitrogen containing dipoles (I) when X₁ is -NH-. One is treating acyl halides or anhydrides with an ethereal solution of diazomethane. Alternatively, one treats methyl ketones with base and ethyl formate to prepare the aldehyde ketone or a salt thereof, and treating this with tosyl azide

There are two preferred ways of preparing the nitrogen containing dipole (I) when X₁ is -O-. One is the *in situ* dehydration of a nitro containing compound by phenylisocyanate. Alternatively, a chloro oxime is treated with an organic base such as TEA or DBU to generate the nitrogen containing dipole (I) *in situ*.

The vinylidene diphosphonates (II) are either known to those skilled in the art or can readily be prepared from known compounds by known methods. See, Tetrahedron 30, 301 (1974) and for R₁ = -H see J. Org. Chem., 51, 3488 (1986). It is preferred that R₁ be -H. The preferred group of geminal phosphonates include the compounds of EXAMPLES 1, 3, 5, 7, 9, 12, 14 and 16-25; more preferred are the compounds of EXAMPLES 1, 3, 5, 7, 9, 14 and 16-25. Another preferred group includes the compounds of EXAMPLES 26-81.

The reaction condensing the nitrogen containing dipoles (I) with the vinylidene diphosphonates (II) to produce the unsaturated geminal phosphonate (III) is well known to those skilled in the art. See, for example, 1,3-Dipolar Cycloaddition Chemistry, ibid. The nitrogen containing dipoles (I) are stirred with the vinylidene diphosphonates (II) in a non-polar solvent such as ether at about 20-25° for about 18-36 hr. When X₁ is -NH-, the unsaturated geminal phosphonate (III) crystallizes out and is obtained by filtration. When X₁ is -O-, the product (III) is obtained by extraction and purification.

The unsaturated geminal phosphonates (III) are readily prepared where R₂$-$1 is -CO-R₂₋₅. These compounds can readily be converted to compounds where R₂$-$1 is -CH(OH)-R₂₋₅ by reaction with a weak reducing agent, such as sodium borohydride, as is well known to those skilled in the art.

The phosphonic esters, either the unsaturated geminal phosphonates (III) are cleaved to the corresponding free acids by methods well known to those skilled in the art, see Tetrahedron Letters 155, (1977). More particularly, the invention uses trimethyl silyl bromide in chloroform followed by treatment with water. The unsaturated geminal phosphonates (III) in the free acid form are readily converted to the corresponding salt forms by reaction with alkali metal hydroxides. There are four acidic -H on the phosphonate, and when X$1 is -NH-, one on the -NH- for a total of five. Therefore, one can have a salt with 1 thru 5 cations. Any salt which is pharmaceutically acceptable is operable. Suitable salts include sodium, potassium, calcium, magnesium, nanganese, copper, gold ethanolamine, diethanolamine, triethanolamine, zinc and THAM. Preferred salts include sodium, potassium, calcium, magnesium and manganese. More preferred are sodium, potassium and calcium.

The unsaturated geminal phosphonates (III) are useful as anti-arthritic agents. The unsaturated geminal phosphonates (III) are useful in humans and lower animals in the treatment of diseases characterized by abnormal phosphate and calcium metabolism and as a treatment of inflammation. These diseases include osteoporosis, Paget's disease, periodontal disease, rheumatoid arthritis, osteo-arthritis, chondrocalcinosis, septic arthritis, neuritis, bursitis, soft tissue mineralization disorders, ankylosing spondylitis, atherosclerosis, multiple myeloma of bone, metastatic bone disease, chronic granulomatous diseases and mitral valve calcification.

The unsaturated geminal phosphonates (III) can be administered orally, parenterally (intramuscularly, intravenously, subcutaneously or intraperitoneally), transdermally or intra-articularly or by suppository. The dose is about 0.1 mg/patient/day to about 1.0 gm/ patient/day.

The unsaturated geminal phosphonates (III) can be used alone or in combination with other pharmaceuticals as is known to those skilled in the art. The exact route of administration, dose, frequency of administration, of a particular unsaturated geminal phosphonate (III), depends on the particular disease or condition, the severity of the disease or condition, the age, general physical condition, weight, other clinical abnomalities, etc., of the particular patient to be treated as is known to those skilled in the art.

For the diseases outlined above intermittent therapy is indicated, as well as continual daily therapy in order to achieve maximum efficacy as is known to those skilled in the art. See, for example, "Long-Term Effects of Dichloromethylene Diphosphonate in Paget's Disease of Bone," P.D. Dumas, et al., J. Clin. Endocrinol. Metab., 54, 837 (1982); "Paget's Disease of Bone Treated in Five Days with AHPrBP(APD) Per Os, " D. Thiebaud, et al., J. Bone Min. Res., 2, 45 (1987); "A Single Infusion of the Bisphosphonate AHPrBP(APD) as Treatment of Paget's Disease of Bone," D. Thiebaud, et al., The Am. J. Med., 85, 207 (1988); "A Double Blind Placebo-controlled Trial of Diphosphonate (APD) Therapy in Rheumatoid Arthritis - Preliminary Results," S.H. Ralston, et al., Calcif. Int., 42, A23 (1988); "Treatment of Hypercalcemia of Malignancy with Intermittent Single Infusions of 3-Amino-1-hydroxypropylidene-1,1-biphosphonate (APD)," D. Rischin, et al., Aust. NZ. J. Med., 18, 736 (1988); "Reduced Morbidity from Skeletal Metastases in aBreast Cancer Patients During Long-Term Bisphosphonate (APD) Treatment," A. Th. van Holten-Verzantvoort, et al., The Lancet, 10-31-87, p. 983; "Sclerosis of Lytic Bone Metastases after Disodium Aminohydroxypropylidene Bisphosphonate (APD) in Patients with Breast Carcinoma," A.R. Morton, et al., British Med. J., 297, 772 (1988); "Two Year Follow-up of Bisphosphonate (APD) Treatment in Steroid Osteoporosis," I.R. Reid, et al., The Lancet 11-12-88, p. 1144.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" in an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parentheses. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom.

Chemical formulas or portions thereof drawn in a linear fashion represent atoms in a linear chain. The symbol "-" in general represents a bond between two atoms in the chain. Thus, CH₃-O-CH₂- CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "=" represents a triple bond, e.g., HC=C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)1-piperazinyl can be represented by -N*-(CH₂)₂-N(C₂H₅)-CH₂-C*H₂.

A rigid cyclic (ring) structure for any compound herein defines an orientation with respect to the plane of the ring for substituents attached to each carbon atom of the rigid cyclic compound. For saturated compounds which have two substituents attached to a carbon atom which is part of a cyclic system, -C(X₁)(X₂)-, the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable R₈ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, the two monovalent variable substituents are α-R₆₋₁:β-R₆₋₂, .... α-R₆₋₉: β-R₆₋₁₀, etc., giving -C(α-R₆₋₁)(β-R₆₋₂)-, .... -(Cα-R₆₋₉)(β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g., due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..."means a lactone in which the carbonyl is bonded to C₂. However, when designated "... Rⱼ and Rᵢ are taken together to form -CO-O-CH₂-CH₂- ..." the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-O-CO- where n is zero, one or two. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

### II. DEFINITIONS

All temperatures are in degrees Centigrade.

TLC refers to thin-layer chromatography.

p-TSA refers to p-toluenesulfonic acid monohydrate.

TEA refers to triethylamine.

DBU refers to 1,8-diazabicyclo[5.4.0]undec-7-ene.

Saline refers to an aqueous saturated sodium chloride solution.

IR refers to infrared specroscopy.

CMR refers to C-13 magnetic resonance spectroscopy, chemical shifts are reported in ppm (δ) downfield from TMS.

NMR refers to nuclear (proton) magnetic resonance spectroscopy, chemical shifts are reported in ppm (δ) downfield from tetramethylsilane.

-φ refers to phenyl (C₆H₅).

MS refers to mass spectrometry expressed as m/e or mass/charge unit. [M + H]⁺ refers to the positive ion of a parent plus a hydrogen atom. EI refers to electron impact. CI refers to chemical ionization. FAB refers to fast atom bombardment.

Ether refers to diethyl ether.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When solvent pairs are used, the ratios of solvents used are volume/volume (v/v).

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### PREPARATION 1 Ethenylidene Bisphosphonic Acid Tetraethyl Ester (II)

Paraformaldehyde (104.2 g) and diethylamine (50.8 g) are combined in methanol (2 l), warmed until clear, then treated with methylene bisphosphonic acid, tetraethyl ester (190.09 g) and refluxed for 18 hrs. The sample is then concentrated, methanol added, the methanol removed by heat and reduced pressure, toluene is added and removed by heat and reduced pressure. The residue is dissolved in toluene (1 l), treated with p-TSA (0.5 g) and refluxed through a Dean Stark trap for 18 hrs. The sample is concentrated under reduced pressure with heat, dissolved in methylene chloride washed twice with water, dried with magnesium sulfate, and concentrated under reduced pressure with heat. The sample is purified by distillation at reduced pressure to give the title compound, bp = 140° (0.6 torr); MS (m/e) 300, 285, 273, 255, 245, 227, 217, 199, 192, 181, 163, 153 and 135; IR (neat) 2984, 2934, 2909, 1651, 1580, 1479, 1444, 1392, 1254, 1166, 1098, 1042, 1025, 974, 855, 813 and 800 cm⁻¹; NMR (CDCl₃) 7.1, 6.7, 4.1 and 1.3 δ.

This compound is known, see EP 221611.

### PREPARATION 2 1-Cyclohexyl-2-diazoethanone (I)

A solution of diazomethane is prepared from N-methyl-N'-nitro-N-nitroso guanidine (12.5 g), potassium hydroxide (50%, 20 ml), and ether (300 ml). TEA (7.7 ml) is added to the diazomethane solution (65%) followed by cyclohexane carboxylic acid chloride (7.4 ml) slowly at 0°. The reaction is stirred at 0° for 1 hr then for 18 hrs at 22°. A porecipitate forms, it is filtered and washed with ether, and the filtrate concentrated. The resulting oil is cooled to -78°, triturated with ether, then warmed to 22° and concentrated with reduced pressure and heat to give the title compound as an oil, NMR (acetone-d₆) 5.4 and 1.4-0.8 δ.

### PREPARATION 3 1-Diazo-3-[2-fluoro(1,1'-biphenyl)-4-yl]-butan-2-one (I)

Flurbiprofen (20.03 g) and thionyl chloride (21 ml) are refluxed for 16 hrs, then the excess thionyl chloride is removed under reduced pressure with heat. The residue is distilled to give the acid chloride which is used without further characterization.

Diazomethane is prepared as in PREPARATION 2 to give approximately 55 mmol. TEA (7.7 ml) is added to the diazomethane solution (300 ml) at -78° under nitrogen, followed by dropwise addition of the flurbiprofen acid chloride (14.4 g) in ether (50 ml). The reaction mixture is stirred for 15 min at -78° then 1 hr at -20°. The reaction is filtered, the filtrate washed with acetic acid (10%), water, saturated sodium bicarbonate solution and saline, then dried with sodium sulfate and concentrated under reduced pressure with heat to give the title compound as an oil, NMR (CDCl₃) 8.0-6.8, 5.1, 3.5 and 1.5 δ.

### PREPARATION 4 1-Diazo-2-butanone (I)

The ethereal diazomethane solution [prepared by adding 12.5 g n-methyl-n-nitro-n-nitroso guanidine to potassium hydroxide (50%, 20 ml) and ether (200 ml) at 0°, stirring for 1 hr, and decanting the ether layer; 65% presumed yield] is treated with TEA 97.7 ml) at 0°, then dropwise with propionyl chloride (5.2 ml) in ether (25 ml). After stirring for 19 hrs, the reaction is filtered through celite and concentrated to give the title compound as an oil.

### PREPARATION 5 2-Diazo-1-[4-nitrophenyl]-ethanone (II)

The ethereal diazomethane solution [prepared by adding 12.5 g n-methyl-n-nitro-n-nitroso guanidine to potassium hydroxide (50%, 20 ml) and ether (200 ml) at 0°, stirring for 1 hr, and decanting the ether layer; 65% presumed yield] is treated with TEA (7.7 ml) at 0°, then dropwise with p-nitrobenzoyl chloride (10.21 ml) in ether (25 ml). After stirring for 19 hrs, the reaction is filtered through celite and concentrated to give the title compound.

### PREPARATION 6 2-Diazo-1-phenylethanone (I)

An ethereal solution of diazomethane is prepared from n-methyl-n-nitro-n-nitroso guanidine (15.72 g) for an estimated yield of 64 mmol. To the ethereal solution (400 ml) at 0° is added triethylamine (9.0 ml, 64 mmol), then dropwise benzoyl chloride (7.4 ml, 64 mmol). The reaction is warmed to 22° and stirred for 20 hrs, then filtered through celite and concentrated. The residue is recrystallized from ether/pentane to give the title compound.

### PREPARATION 7 2-Diazo-1-phenylethanone (I)

Sodium hydride (50% in oil, 0.53 g) is suspended in ether (20 ml), cooled to 0°, then treated dropwise with a mixture of acetophenone 1.2 ml) and ethyl formate (0.90 ml). The reaction is stirred 1 hr at 0° then overnight at 22°. The precipitate is filtered and washed well with ether to give a solid. The solid is suspended in ethanol (10 ml), cooled to 0°, then treated dropwise with tosyl azide (0.97 g). The reaction is stirred for 3 hrs, concentrated, dissolved in IN sodium hydroxide and extracted with ether. The ether is washed with water, dried with sodium sulfate and concentrated to give the title compound.

### PREPARATION 8 2-Chloro-2-oximino-1-phenylethanone (I)

Phenacyl chloride (15.4 g) is dissolved in ether (100 ml) and hydrogen chloride gas is bubbled through the solution. Isoamyl nitrite (13.4 ml) is added in 0.5 to 1 ml portions over a 30 min period. Hydrochloric acid is continuously bubbled through the solution for an additional 15 min. The reaction is concentrated under reduced pressure to an oil, stored under vaccuum in the presence of sulfuric acid, sodium hydroxice, and calcium chloride. The oil is crystallized from toluene and carbon tetrachloride to give the title compound.

### EXAMPLE 1 [5-Benzoyl-2,4-dihydro-3H-pyrazol-3-ylidene]bis-phosphonic acid tetraethyl ester (III)

Ethenylidene bisphosphonic acid tetraethyl ester (II, PREPARATION 1, 6.00 g) in ether (20 ml) is treated with solid 2-diazo-1-phenylethanone (2.92 g) and stirred at 22°for 20 hrs. The precipitate is collected and washed with ether to give the title compound. An analytical sample is obtained by recrystallization from methylene chloride/hexane, mp 133.5-134°; MS (m/e) 446, 327, 309, 281, 271, 253, 243, 215, 175; IR (mineral oil mull) 3183, 1631, 1600, 1578, 1548, 1433, 1260, 1242, 1161, 1067, 1043, 1020, 1001, 995, 976 and 962 cm⁻¹; NMR (CDCl₃) 8.10, 7.50, 6.90, 4.27, 3.69 and 1.34 δ.

### EXAMPLE 2 [5-Benzoyl-2,4-dihydro-3H-pyrazol-3-ylidene]bis-phosphonic acid, disodium salt (III)

[5-Benzoyl-2,4-dihydro-3H-pyrazol-3-ylidene]bis-phosphonic acid tetraethyl ester (III, EXAMPLE 1, 2.07 g) in chloroform (10 ml) is treated with bromotrimethylsilane (3.7 ml), heated to 40° for 5 hrs, and then concentrated under reduced pressure with heat. The residue is diluted with ethyl acetate and water, then stirred for 30 min. The layers are separated and the aqueous layer is treated with activated charcoal, filtered through celite, and freeze dried to give the free acid. The crude acid is then converted to the disodium salt. The acid (1.43 g) in methanol (15 ml) is warmed to dissolve most of the solid, filtered, then treated with a sodium methoxide in methanol solution (25%, 2.0 ml). The reaction mixture is cooled, filtered, and the precipitate air dried to give the title compound, mp dec > 170°; MS (m/e) 379, 357, 335, 315, 297, 281, 267, 253 and 239; RI (mineral oil mull) 3209, 1599, 1572, 1267, 1178, 1098, 1029, 1002 and 914 cm⁻¹; NMR (D₂O) 7.9-7.45 and 3.53 δ.

### EXAMPLE 3 [5-(Cyclohexylcarbonyl)-2,4-dihydro-3H-pyrazol-3-ylidene]bisphosphonic acid tetraethyl ester (III)

1-Cyclohexyl-2-diazoethanone (I, PREPARATION 2, 3.1 g) and ethenylidene bisphosphonic acid tetraethyl ester (II, PREPARATION 1, 6.00 g) are stirred in ether (20 ml) for 24 hrs at 22°. The precipitate is filtered, washed with ether, then recrystallized from ethyl acetate to give the title compound, mp 166-165°; MS (m/e) 452, 341, 315, 287, 259, 241, 223 and 205; IR (mineral oil mull) 3186, 1656, 1552, 1449, 1267, 1253, 1241, 1162, 1041, 1019, 995 and 969 cm⁻¹; NMR (CDCl₃) 6.77, 4.24, 3.45, 3.24 and 1.9-1.2 δ.

### EXAMPLE 4 [5-(Cyclohexylcarbonyl)-2,4-dihydro-3H-pyrazol-3-ylidene]bisphosphonic acid dipotassium salt (III)

Following the general procedure of EXAMPLE 2 and making non-critical variations but starting with [5-(Cyclohexylcarbonyl)-2,4-dihydro-3H-pyrazol-3-ylidene]bisphosphonic acid tetraethyl ester (III, EXAMPLE 3, 2.08 g) and using potassium hydroxide in methanol, the title compound is obtained, mp dec > 150°; MS (m/e) 495, 455, 417, 379 and 341; IR (mineral oil mull) 3177, 1630, 1547, 1350, 1166, 1138, 1076, 1058 and 972 cm⁻¹; NMR (CDCl₃, D₂O) 3.31, 3.11, 1.69 and 1.30 δ.

### EXAMPLE 5 [5-[2-(2-Fluoro[1,1'-biphenyl]-4-yl)-1-oxopropyl]-2,4-dihydro-3H-pyrazol-3-ylidene]bisphosphonic acid tetraethyl ester (III)

Ethenylidene bisphosphonic acid tetraethyl ester (II, PREPARATION 1, 11.1 g) and 1-diazo-3-[2-fluoro(1,1'-biphenyl)-4-yl]-butan-2-one (I, PREPARATION 3, 10.0 g) are stirred for 18 hrs in ether (40 ml). A need crystal from an earlier reaction is added to the mixture, whereupon the reaction sets up into a solid mass. The precipitate is filtered, then recrystallized from methylene chloride/ hexane (45(55) to give the title compound, mp 109-110°; MS (m/e) 568, 431, 403, 385, 341, 313, 301 and 199; IR (mineral oil mull) 3179, 1656, 1557, 1265, 1246, 1033, 1009, 993, 976 and 965 cm⁻¹; NMR (CDCl₃) 7.4, 7.2, 6.91, 4.81, 4.4, 4.05, 3.4, 1.49, 1.34, 1.16 and 1.08 δ; CMR (CDCl₃) 195.1, 162, 158, 149, 142, 136, 130.7, 128.8, 128.5, 127.6, 123.9, 115.8, 115.6, 64.3, 63.6, 46.1, 36.4, 17.7, 16.5, 16.2 and 16.1 δ.

### EXAMPLE 6 [5-[2-(2-Fluoro[1,1'-biphenyl]-4-yl)-1-oxopropyl]-2,4-dihydro-3H-pyrazol-3-ylidene]bisphosphonic acid dipotassium salt with ethanol (III)

Following the general procedure of EXAMPLE 2 and making non-critical variations but starting with p5-[2-(2-fluoro[1,1'-biphenyl]-4-yl)-1-oxopropyl]-2,4-dihydro-3H-pyrazol-3-ylidene]bisphosphonic acid tetraethyl ester (III, EXAMPLE 5, 4.00 g) and using potassium hydroxide in ethanol, the title compound is obtained, mp dec > 140°; MS (m/e) 533, 495, 451, 413 and 375; IR (mineral oil mull) 1651, 1623, 1581, 1561, 1483, 1219, 1074 and 1011 cm⁻¹; NMR (CDCl₃) 7.15, 3.68, 3.38, 1.35 and 1.20 δ.

### EXAMPLE 7 [3-Methyl-5(4H)-isoxazolyidene]bisphosphonic acid tetraethyl ester (III)

Nitroethane (2.4 ml), TEA (6 drops) and toluene (9 ml) are added in one portion to a mixture of ethenylidene bisphosphonic acid tetraethyl ester (II, PREPARATION 1, 10.0 g) and phenyl isocyanate (6.6 ml) in toluene (16 ml). The mixture is stirred at 20-25° for 30 min then at reflux for 4 hrs. The reaction is then cooled, filtered and the precipitate washed with ethyl acetate. The filtrate is concentrated under reduced pressure with heat. Column chromatography eluting with acetone/methylene chloride (1/1) gives the title compound, NMR (CDCl₃) 4.2, 3.5, 2.1 and 1.3 δ.

### EXAMPLE 8 [3-Methyl-5(4H)-isoxazolylidene]bisphosphonic acid disodium salt (III)

[3-Methyl-5(4H)-isoxazolylidene]bisphosphonic acid tetraethyl ester (III, EXAMPLE 7, 3.00 g) is dissolved in chloroform (20 ml), treated with bromotrimethyl silane (6.6 ml) and heated to 40° for 4 hrs, then under reduced pressure with heat. The residue is taken up in ethyl acetate and water and stirred for 20 min. The aqueous layer is separated, washed with ethyl acetate, then freeze dried to give (3-methyl-5(4H)-isoxazolylidene)bisphosphonic acid (III).

The free acid, [3-methyl-5(4H)-isoxazolylidene]bisphosphonic acid (III, 1.756 g) is dissolved in methanol (20 ml), treated with Darco, and filtered through celite. The filtrate is treated with 25% sodium methoxide/methanol (25/75, 3.2 ml), stirred for 5 min, then filtered to give the title compound, mp dec > 250°; MS (m/e) 290, 268, 246 and 211; IR (mineral oil mull) 3300, 2345, 1648, 1548, 1330, 1191, 1110 and 970 cm⁻¹; NMR (D₂O) 3.45 and 1.89 δ.

### EXAMPLE 9 [3-Phenyl-5(4H)-isoxazolylidene]bisphosphonic acid tetraethyl ester (III)

Benzaldehyde-wyn-oxime (3.83 g) is dissolved in methylene chloride (30 ml), treated with a steady stream of chlorine gas for 5 min, then under reduced pressure with heat. The residue is again dissolved in methylene chloride (30 ml), treated with TEA (8.8 ml), and stirred for 5 min. Ethenylidene bisphosphonic acid tetraethyl ester (II, PREPARATION 1, 9.00 g) in methylene chloride (10 ml) is added and the reaction stirred well for 1 hr. The solution is then washed with hydrochloric acid (IN), saturated sodium bicarbonate and saline, dried with magnesium sulfate, and concentrated under reduced pressure with heat. Column chromatography eluting with hexane/acetone (7/3) and pooling the appropriate fraction gives the title compound as an oil, NMR (CDCl₃) 7.6, 7.3, 4.3, 3.9 aand 1.3 δ.

### EXAMPLE 10 [3-Phenyl-5(4H)-isoxazolylidene]bisphosphonic acid P-P'-diethyl ester disodium salt (III)

[3-Phenyl-5(4H)-isoxazolylidene]bisphosphonic acid tetraethyl ester (III, 9, 4.19 g) is dissolved in methyl ethyl ketone (10 ml) and heated to reflux with sodium iodide (3.00 g) for 4 hrs. Filtered, resuspended in methanol and filtered again to give the title compound, MS (m/e) 408, 386, 362, 327 and 298; IR (mineral oil mull) 3415, 1679, 1601, 1572, 1366, 1223, 1204, 1124, 1081, 1076 and 1051 cm⁻¹; NMR (CDCl₃) δ; NMR (CDCl₃) 7.7, 7.4, 4.4, 4.0 and 1.3 δ.

### EXAMPLE 11 [3-Phenyl-5(4H)-isoxazolylidene]bisphosphonic acid monopotassium salt (III)

The diphosphonate, [3-phenyl-5(4H)-isoxazolylidene]bisphosphonic acid tetraethyl ester (III, EXAMPLE 9, 2.10 g) in chloroform (10 ml) is treated with bromotrimethylsilane (4.0 ml) and heated to 50-60° for 5 hrs. The reaction is concentrated under reduced pressure with heat, taken up in ethyl acetate, extracted with water, and the aqueous fractions are filtered and freeze dried to give (3-phenyl-5(4H)-isoxazolylidene)bisphosphonic acid.

The free acid, [3-phenyl-5(4H)-isoxazolylidene]bisphosphonic acid, is then dissolved in ethanol (15 ml), treated with a solution of potassium hydroxide (560 mg, 10 mmol) in ethanol (10 ml), the precipitate collected and washed with ether to give the title compound, mp dec > 250°; MS (m/e) 422, 384, 346 and 308; IR (mineral oil mull) 3054, 3029, 2317, 1609, 1572, 1497, 1206 and 1068 cm⁻¹; NMR (D2O) 3.94 δ.

### EXAMPLE 12 [2,4-Dihydro-5-(1-oxopropyl)-3H-pyrazol-3-ylidene]bis-phosphonic acid tetraethyl ester (III)

1-Diazo-2-butanone (I, PREPARATION 4, 3.1 g) in ether (50 ml) is treated with ethenylidene bisphosphonic acid tetraethyl ester (II, PREPARATION 1, 9.5 g), stirred at 20-25° overnight, and concentrated. The concentrate is crystallized twice from methylene chloride/SSB to give the title compound, mp 96-98°; MS (m/e) 398, 261, 233 and 205; IR (mineral oil mull) 3210, 0664, 1265, 1245, 1046, 1024, 997 and 983 cm⁻¹; NMR (CDCl₃) 6.92, 4.14-4.33, 3.48, 2.82, 1.30-1.36 and 1.12 δ; CMR (CDCl₃) 196, 149, 66, 64, 36, 31, 16 and 8 δ.

### EXAMPLE 13 [2,4-Dihydro-5-(1-oxopropyl)-3H-pyrazol-3-ylidene]bis-phosphonic acid (III)

[2,4-Dihydro-5-(1-oxopropyl)-3H-pyrazol-3-ylidene]bisphosphonic acid tetraethyl ester (III, EXAMPLE 12, 1.56 g) and bromotrimethylsilane (2.6 ml) in chloroform (20 ml) are stirred at 50° for 4 hrs, then concentrated. The concentrate is diluted with water and ethyl acetate, shaken, and the aqueous layer separated and freeze dried to give the title compound, mp 148° foamed, IR (mineral oil mull) 3325, 1597, 1532, 1430, 1189, 1173, 1060, 1019 and 937 cm⁻¹; NMR (D₂O) 3.40, 2.84 and 1.07 δ.

### EXAMPLE 14 [2,4-Dihydro-5-(nitrobenzoyl)-3H-pyrazol-3-ylidene]-bisphosphonic acid tetraethyl ester (III)

2-Diazo-1-[4-nitrophenyl]-ethanone (I, PREPARATION 5, 2.7 g) in ether (30 ml) is treated with ethenylidene bisphosphonic acid tetraethyl ester (II, PREPARATION 1, 4.6 g) stirred at 20-25° for 48 hrs, filtered and the solid washed with ether. The solid is crystallized twice from methylene chloride/SSB to give the title compound, mp 111-112°; IR (mineral oil mull) 3185, 1635, 1555, 1515, 1353, 1253, 1237, 1056, 1042, 1017, 994 and 603 cm⁻¹; CMR (CDCl₃) 185, 150, 148, 141, 130, 123, 65, 64, 37 and 16 δ.

### EXAMPLE 15 [2,4-Dihydro-5-(4-nitrobenzoyl)-3H-pyrazol-3-ylidene]-bisphosphonic acid (III)

Following the general procedure of EXAMPLE 13 and making non-critical variation but starting with [2,4-dihydro-5-(4-nitrobenzoyl)-3H-pyrazol-3-ylidene]bisphosphonic acid tetraethyl ester (III, EXAMPLE 14, 2.4 g) the title compound is obtained, mp 180° dec; NMR (CDCl₃) 8.23, 7.98 and 3.64 δ; CMR (D₂O) 188, 149, 145, 142, 130, 123, 68 and 35 δ.

### EXAMPLE 16 [3-Benzoyl-5(4H)isoxazolylidene]bisphosphonic acid tetraethyl ester (III)

A solution of 2-chloro-2-oximino-1-phenylethanone (I, PREPARATION 8, 0.92 g) and ethenylidene bisphosphonic acid tetraethyl ester (II, PREPARATION 1, 1.50 g) in methylene chloride (5 ml) are treated with triethylamine (1.0 ml) and stirred for 20 hrs. The reaction is diluted with ethyl acetate, washed with water, hydrochloric acid (IN) and saturated sodium bicarbonate, dried with magnesium sulfate and concentrated under reduced pressure. Two reactions of identical size are chromatographed over a silica gel column eluting with ethyl acetate. The appropriate fractions are pooled and concentrated to give the title compound, NMR (CDCl₃) 8.11, 8.54, 7.40, 4.23, 3.90 and 1.28 δ.

### EXAMPLES 17 - 25

Following the general procedure of EXAMPLES 1 and 3 and making non-critical variations but starting with the nitrogen containing dipoles (I) 17A, 18A, ... 25A, the corresponding unsaturated geminal phosphonates (III) of EXAMPLES 17B, 18B, ... 25B, are obtained.

The nitrogen containing dipoles (I)
17A 1-(4'-chlorophenyl)-2-diazoethanone
18A 1-(2',4'-dichlorophenyl)-2-diazoethanone
19A 1-diazo-3,3-dimethylbutanone
20A 1-(2'-fluorophenyl)-2-diazoethanone
21A 1-(4'-methoxyphenyl)-2-diazoethanone
22A 1-(4'-methylphenyl)-2-diazoethanone
23A 1-(3'-methylphenyl)-2-diazoethanone
24A 1-(3,-fluorophenyl)-2-diazoethanone
25A 1-(2'-methylphenyl)-2-diazoethanone

produce the unsaturated geminal phosphonates (III)
17B [5-(4'-chlorobenzoyl)-2,4-dihydro-3H-pyrazol-3-ylidene]-bisphosphonic acid tetraethyl ester, mp 101-102°.
18B [5-(2',4'-dichlorobenzoyl)-2,4-dihydro-3H-pyrazol-3-ylidene]-bisphosphonic acid tetraethyl ester, mp 155-156°.
19B [5-(2,2-dimethyl-1-oxopropyl)-2,4-dihydro-3H-pyrazol-3-ylidene]-bisphosphonic acid tetraethyl ester, mp 106-107°.
20B [5-(2'-fluorobenzoyl)-2,4-dihydro-3H-pyrazol-3-ylidene]-bisphosphonic acid tetraethyl ester, mp 185-186°.
21B [5-(4'-methoxybenzoyl)-2,4-dihydro-3H-pyrazol-3-ylidene]-bisphosphonic acid tetraethyl ester, mp 115-116°.
22B [5-(4'-methylbenzoyl)-2,4-dihydro-3H-pyrazol-3-ylidene]-bisphosphonic acid tetraethyl ester, mp 116-117°.
23B [5-(3'-methylbenzoyl)-2,4-dihydro-3H-pyrazol-3-ylidene]-bisphosphonic acid tetraethyl ester, mp 91-92°.
24B [5-(3'-fluorobenzoyl)-2,4-dihydro-3H-pyrazol-3-ylidene]-bisphosphonic acid tetraethyl ester, mp 104-105°.
25B [5-(2'-methylbenzoyl)-2,4-dihydro-3H-pyrazol-3-ylidene]-bisphosphonic acid tetraethyl ester, mp 162-163°.

### EXAMPLES 26 - 81

Following the general procedure of EXAMPLES 1 and 3 and making non-critical variations but- starting with the nitrogen containing dipoles (I) 26A, ... 81A, the corresponding unsaturated geminal phosphonates (III) of EXAMPLES 26B, ... 81B are obtained.

The nitrogen containing dipoles (I) 26A, ... 81A are prepared following the general procedure of PREPARATIONS 5 or 6.
26A 1-(2'-bromophenyl)-2-diazoethanone
27A 1-(3'-bromophenyl)-2-diazoethanone
28A 1-(4'-bromophenyl)-2-diazoethanone
29A 1-(2'-chlorophenyl)-2-diazoethanone
30A 1-(3'-chlorophenyl)-2-diazoethanone
31A 1-(3',4'-dichlorophenyl)-2-diazoethanone
32A 1-(3',5'-dichlorophenyl)-2-diazoethanone
33A 1-(2',6'-dichlorophenyl)-2-diazoethanone
34A 1-(2',3'4'-trichlorophenyl)-2-diazoethanone
35A 1-(4'-fluorophenyl)-2-diazoethanone
36A 1-(2',3',4',5',6'-pentafluorophenyl)-2-diazoethanone
37A 1-(2'-methoxyphenyl)-2-diazoethanone
38A 1-(3'-methoxyphenyl)-2-diazoethanone
39A 1-(2',4'-dimethoxyphenyl)-2-diazoethanone
40A 1-(3',5'-dimethoxyphenyl)-2-diazoethanone
41A 1-(2'-chloro-4'-methoxyphenyl)-2-diazoethanone
42A 1-(4'-chloro-2'-methoxyphenyl)-2-diazoethanone
43A 1-(3'-chloro-4'-methoxyphenyl)-2-diazoethanone
44A 1-(4'-chloro-3'-methoxyphenyl)-2-diazoethanone
45A 1-(2'-ethoxyphenyl)-2-diazoethanone
46A 1-(3'-ethoxyphenyl)-2-diazoethanone
47A 1-(4'-ethoxyphenyl)-2-diazoethanone
48A 1-(2'-phenoxyphenyl)-2-diazoethanone
49A 1-(3'-phenoxyphenyl)-2-diazoethanone
50A 1-(4'-phenoxyphenyl)-2-diazoethanone
51A 1-(2'-methylthiophenyl)-2-diazoethanone
52A 1-(3'-methylthiophenyl)-2-diazoethanone
53A 1-(4'-methylthiophenyl)-2-diazoethanone
54A 1-(2'-chloro-4'-methylphenyl)-2-diazoethanone
55A 1-(4'-chloro-2'-methylphenyl)-2-diazoethanone
56A 1-(3'-chloro-4'-methylphenyl)-2-diazoethanone
57A 1-(4'-chloro-3'-methylphenyl)-2-diazoethanone
58A 1-(2'-ethylphenyl)-2-diazoethanone
59A 1-(3'-ethylphenyl)-2-diazoethanone
60A 1-(4'-ethylphenyl)-2-diazoethanone
61A 1-(4'-tert butylphenyl)-2-diazoethanone
62A 1-(2',4'-dimethylphenyl)-2-diazoethanone
63A 1-(3',5'-dimethylphenyl)-2-diazoethanone
64A 1-(2'-phenylphenyl)-2-diazoethanone
65A 1-(3'-phenylphenyl)-2-diazoethanone
66A 1-(4'-phenylphenyl)-2-diazoethanone
67A 1-(4'-inophenyl)-2-diazoethanone
68A 1-(4'-dimethylaminophenyl)-2-diazoethanone
69A 1-(4'-diethylaminophenyl)-2-diazoethanone
70A 1-(3'-trifluoromethylphenyl)-2-diazoethanone
71A 1-(1'-naphthoyl)-2-diazoethanone
72A 1-(2'-naphthoyl)-2-diazoethanone
73A 1-(6'-quinolinoyl)-2-diazoethanone
74A 1-(8'-quinolinoyl)-2-diazoethanone
75A 1-(2'-thienylcarbonyl)-2-diazoethanone
76A 1-(2'-pyridylcarbonyl)-2-diazoethanone
77A 1-(nicotinoyl)-2-diazoethanone
78A 1-(4-pyridylcarbonyl)-2-diazoethanone
79A 1-(cyclopropanoyl)-2-diazoethanone
80A 1-(cyclobutanoyl)-2-diazoethanone
81A 1-(cyclopentanoyl)-2-diazoethanone
82A 1-(9'-anthraconyl)-2-diazoethanone
83A 1-(3',5'-difluorophenyl)-2-diazoethanone

produce the unsaturated geminal phosphonates (III)
26B [5'-(2'-bromobenzoyl)-2,4-dihydro-3H-pyrazol-3-ylidene]-bisphosphonic acid tetraethyl ester
27B [5'-(3'-bromobenzoyl)-2,4-dihydro-3H-pyrazol-3-ylidene]-bisphosphonic acid tetraethyl ester
28B [5'-(4'-bromobenzoyl)-2,4-dihydro-3H-pyrazol-3-ylidene]-bisphosphonic acid tetraethyl ester, mp 104-105°
29B [5'-(2'-chlorobenzoyl)-2,4-dihydro-3H-pyrazol-3-ylidene]-bisphosphonic acid tetraethyl ester
30B [5'-(3'-chlorobenzoyl)-2,4-dihydro-3H-pyrazol-3-ylidene]-bisphosphonic acid tetraethyl ester
31B [5'-(3',4'-dichlorobenzoyl)-2,4-dihydro-3H-pyrazol-3-ylidene]bisphosphonic acid tetraethyl ester
32B [5'-(3',5'-dichlorobenzoyl)-2,4-dihydro-3H-pyrazol-3-ylidene]bisphosphonic acid tetraethyl ester
33B [5'-(2',6'-dichlorobenzoyl)-2,4-dihydro-3H-pyrazol-3-ylidene]bisphosphonic acid tetraethyl ester
34B [5'-(2',3',4'-trichlorobenzoyl)-2,4-dihydro-3H-pyrazol-3-ylidene]bisphosphonic acid tetraethyl ester, mp 171-172°.
35B [5'-(4'-fluorobenzoyl)-2,4-dihydro-3H-pyrazol-3-ylidene]-bisphosphonic acid tetraethyl ester
36B [5'-(2',3',4',5',6'-pentafluorobenzoyl)-2,4-dihydro-3H-pyrazol-3-ylidene]bisphosphonic acid tetraethyl ester
37B [5'-(2'-methoxybenzoyl)-2,4-dihydro-3H-pyrazol-3-ylidene]-bisphosphonic acid tetraethyl ester
38B [5'-(3'-methoxybenzoyl)-2,4-dihydro-3H-pyrazol-3-ylidene]-bisphosphonic acid tetraethyl ester, mp 90-91°
39B [5'-(2',4'-dimethoxybenzoyl)-2,4-dihydro-3H-pyrazol-3-ylidene]bisphosphonic acid tetraethyl ester, mp 120°
40B [5'-(3',5'-dimethoxybenzoyl)-2,4-dihydro-3H-pyrazol-3-ylidene]bisphosphonic acid tetraethyl ester
41B [5'-(2'-chloro-4'-methoxybenzoyl)-2,4-dihydro-3H-pyrazol-3-ylidene]bisphosphonic acid tetraethyl ester
42B [5'-(4'-chloro-2'-methoxybenzoyl)-2,4-dihydro-3H-pyrazol-3-ylidene]bisphosphonic acid tetraethyl ester
43B [5'-(3'-chloro-4'-methoxybenzoyl)-2,4-dihydro-3H-pyrazol-3-ylidene]bisphosphonic acid tetraethyl ester
44B [5'-(4'-chloro-3'-methoxybenzoyl)-2,4-dihydro-3H-pyrazol-3-ylidene]bisphosphonic acid tetraethyl ester
45B [5'-(2'-ethoxybenzoyl)-2,4-dihydro-3H-pyrazol-3-ylidene]-bisphosphonic acid tetraethyl ester
46B [5'-(3'-ethoxybenzoyl)-2,4-dihydro-3H-pyrazol-3-ylidene]-bisphosphonic acid tetraethyl ester
47B [5'-(4'-ethoxybenzoyl)-2,4-dihydro-3H-pyrazol-3-ylidene]-bisphosphonic acid tetraethyl ester
48B [5'-(2'-phenoxybenzoyl)-2,4-dihydro-3H-pyrazol-3-ylidene]-bisphosphonic acid tetraethyl ester
49B [5'-(3'-phenoxybenzoyl)-2,4-dihydro-3H-pyrazol-3-ylidene]-bisphosphonic acid tetraethyl ester
50B [5'-(4'-phenoxybenzoyl)-2,4-dihydro-3H-pyrazol-3-ylidene]-bisphosphonic acid tetraethyl ester
51B [5'-(2'-methylthiobenzoyl)-2,4-dihydro-3H-pyrazol-3-ylidene]bisphosphonic acid tetraethyl ester
52B [5'-(3'-methylthiobenzoyl)-2,4-dihydro-3H-pyrazol-3-ylidene]bisphosphonic acid tetraethyl ester
53B [5'-(4'-methylthiobenzoyl)-2,4-dihydro-3H-pyrazol-3-ylidene]bisphosphonic acid tetraethyl ester
54B [5'-(2'-chloro-4'-methylbenzoyl)-2,4-dihydro-3H-pyrazol-3-ylidene]bisphosphonic acid tetraethyl ester
55B [5,-(4'-chloro-2'-methylbenzoyl)-2,4-dihydro-3H-pyrazol-3-ylidene]bisphosphonic acid tetraethyl ester
56B [5'-(3'-chloro-4'-methylbenzoyl)-2,4-dihydro-3H-pyrazol-3-ylidene]bisphosphonic acid tetraethyl ester
57B [5'-(4'-chloro-3'-methylbenzoyl)-2,4-dihydro-3H-pyrazol-3-ylidene]bisphosphonic acid tetraethyl ester
58B [5'-(2'-ethylbenzoyl)-2,4-dihydro-3H-pyrazol-3-ylidene]-bisphosphonic acid tetraethyl ester
59B [5'-(3'-ethylbenzoyl)-2,4-dihydro-3H-pyrazol-3-ylidene]-bisphosphonic acid tetraethyl ester
60B [5'-(4'-ethylbenzoyl)-2,4-dihydro-3H-pyrazol-3-ylidene]-bisphosphonic acid tetraethyl ester
61B [5'-(4'-tert-butylbenzoyl)-2,4-dihydro-3H-pyrazol-3-ylidene]bisphosphonic acid tetraethyl ester
62B [5'-(2',4'-dimethylbenzoyl)-2,4-dihydro-3H-pyrazol-3-ylidene]bisphosphonic acid tetraethyl ester
63B [5'-(3',5'-dimethylbenzoyl)-2,4-dihydro-3H-pyrazol-3-ylidene]bisphosphonic acid tetraethyl ester
64B [5'-(2'-phenylbenzoyl)-2,4-dihydro-3H-pyrazol-3-ylidene]-bisphosphonic acid tetraethyl ester
65B [5'-(3'-phenylbenzoyl)-2,4-dihydro-3H-pyrazol-3-ylidene]-bisphosphonic acid tetraethyl ester
66B [5'-(4'-phenylbenzoyl)-2,4-dihydro-3H-pyrazol-3-ylidene]-bisphosphonic acid tetraethyl ester
67B [5'-(4'-morpholinobenzoyl)-2,4-dihydro-3H-pyrazol-3-ylidene]bisphosphonic acid tetraethyl ester
68B [5'-(4'-dimethylaminobenzoyl)-2,4-dihydro-3H-pyrazol-3-ylidene]bisphosphonic acid tetraethyl ester
69B [5'-(4'-diethylaminobenzoyl)-2,4-dihydro-3H-pyrazol-3-ylidene]bisphosphonic acid tetraethyl ester
70B [5'-(3'-trifluoromethylbenzoyl)-2,4-dihydro-3H-pyrazol-3-ylidene]bisphosphonic acid tetraethyl ester, mp 94-95°
71B [5'-(1'-naphthoyl)-2,4-dihydro-3H-pyrazol-3-ylidene]bisphosphonic acid tetraethyl ester, mp 176-177°
72B [5'-(2'-naphthoyl)-2,4-dihydro-3H-pyrazol-3-ylidene]bisphosphonic acid tetraethyl ester
73B [5'-(6'-quinolinoyl)-2,4-dihydro-3H-pyrazol-3-ylidene]bisphosphonic acid tetraethyl ester
74B [5'-(8'-quinolinoyl)-2,4-dihydro-3H-pyrazol-3-ylidene]bisphosphonic acid tetraethyl ester
75B [5'-(2'-thienylcarbonyl)-2,4-dihydro-3H-pyrazol-3-ylidene]-bisphosphonic acid tetraethyl ester
76B [5'-(2'-pyridylcarbonyl)-2,4-dihydro-3H-pyrazol-3-ylidene]-bisphosphonic acid tetraethyl ester
77B [5'-(nicotinoyl)-2,4-dihydro-3H-pyrazol-3-ylidene]bisphosphonic acid tetraethyl ester
78B [5'-(4'-pyridylcarbonyl)-2,4-dihydro-3H-pyrazol-3-ylidene]-bisphosphonic acid tetraethyl ester
79B [5'-(cyclopropanoyl)-2,4-dihydro-3H-pyrazol-3-ylidene]bisphosphonic acid tetraethyl ester, mp 133-134°
80B [5'-(cyclobutanoyl)-2,4-dihydro-3H-pyrazol-3-ylidene]bisphosphonic acid tetraethyl ester
81B [5'-(cyclopentanoyl)-2,4-dihydro-3H-pyrazol-3-ylidene]bisphosphonic acid tetraethyl ester
82B [5'-(9-'anthracenoyl)-2,4-dihydro-3H-pyrazol-3-ylidene]bisphosphonic acid tetraethyl ester, mp 201-202°
83B [5'-(3',5'-difluorobenzoyl)-2,4-dihydro-3H-pyrazol-3-ylidene]bisphosphonic acid tetraethyl ester, 105-106°.

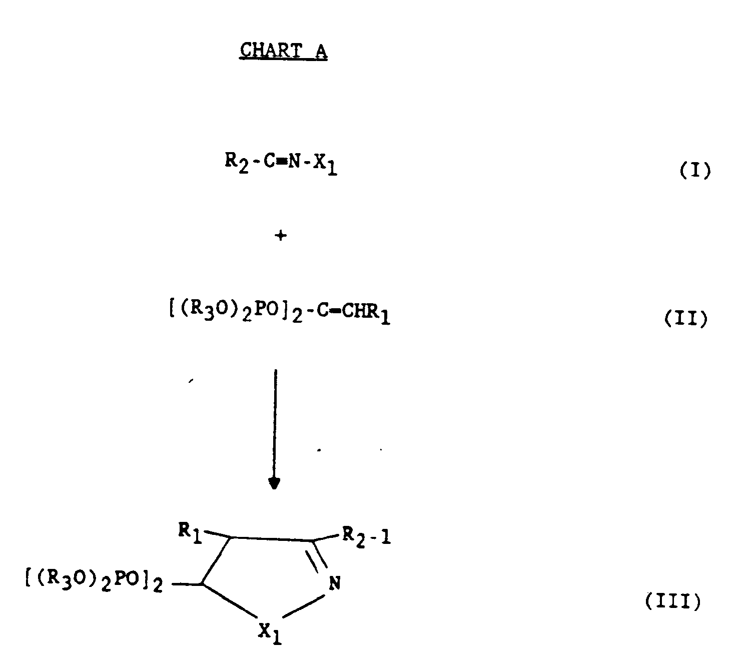

## Claims

1. An unsaturated geminal phosphate of formula (III) 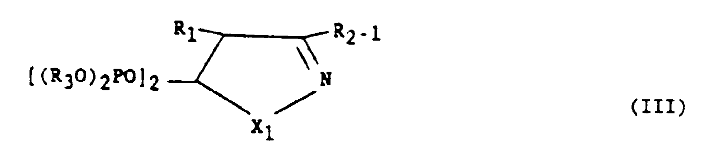 where
X₁ is -O-, -NH- or -N-metal where metal is sodium, potassium, calcium, magnesium, copper, zinc, barium, silver and gold;
R₁ is -H, C₁-C₄ alkyl, C₃-C₇ cycloalkyl, -φ optionally substituted with 1 through 5 -F, -Cl, -Br, -I, -CF₃, C₁-C₆ alkyl, C₁-C₆ cycloalkyl, C₁-C₄ alkoxy or C₁-C₄ alkylthio;
R₂₋₁ is
C₁-C₆ alkyl,
C₃-C₇ cycloalkyl,
-φ optionally substituted with 1 through 5 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
-CH(OH)-R₂₋₅ where R₂₋₅ is
(A) C₁-C₁₀ alkyl,
(B) C₃-C₇ cycloalkyl,
(C) -φ optionally substituted with 1 or 2 -φ or 1 through 5 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(D) 2- and 3-furanyl optionally substituted with 1 through 3 -F, -Cl, -Br, -I, -NO₂, -CN, -CF₃, C₁-C₆ alkyl, C₃-C₇ cycloalkyl, C₁-C₄ alkoxy, -O-φ, C₁-C₄ alkylthio,
(E) 2-, 4- and 5-pyrimidyl optionally substituted with 1 through 3 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(F) 2-, 3- and 4-pyridinyl optionally substituted with 1 through 4 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(G) 2- and 3-thiophene optionally substituted with 1 through 3 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(H) 1- and 2-naphthalene optionally substituted with 1 through 7 -F, -Cl, -Br, -I, -NO₂, -CN, -CF₃, C₁-C₆ alkyl, C₃-C₇ cycloalkyl, C₁-C₄ alkoxy, C₁-C₄ alkylthio,
(I) 2-, 3-, 4-, 6-, 7- and 8-quinoline,
(J) 1-, 3-, 4-, 6-, 7- and 8-isoquinoline,
(K) 2-, 3-, 4-, 5-, 6- and 7-benzothiophene,
(L) 2-, 3-, 4-, 5-, 6- and 7-benzofuran,
(M) -NR₂₋₆R₂₋₇ where R₂₋₆ and R₂₋₇ are the same or different and are C₁-C₄ alkyl,
-φ,
-CO-R₂₋₈ wherein R₂₋₈ is C₁-C₄ alkyl or -φ optionally substituted with 1 -CH₃,
-SO₂-R₂₋₈ where R₂₋₈ is as defined above and where R₂₋₆ and R₂₋₇ are taken together with the attached nitrogen atom to form a 4 through 8 member heterocyclic ring containing a nitrogen, oxygen or sulfur heteroatom and 0 thru 3 double bonds,
-CO-R₂₋₅ where R₂₋₅ is as defined above;
R₃ is -H, C₁-C₆ alkyl, -φ and pharmaceutically acceptable salts thereof.

2. An unsaturated geminal phosphonate (III) according to claim 1 where X₁ is -NH-.

3. An unsaturated geminal phosphonate (III) according to claim 1 where R₁ is -H.

4. An usaturated geminal phosphonate (III) according to claim 1 where R₂₋₁ is -CO-R₂₋₅ and -CH(OH)-R₂₋₅.

5. An unsaturated geminal phosphonate (III) according to claim 1 where R₂₋₁ is -CO-φ optionally substituted with -F, -Cl, -Br, -I, -CN, -CF₃, C₁-C₄ alkoxy, C₁-C₄ alkyl, -N(CH₃)₂, -N(CH₂CH₃)₂, morpholino, 1- and 2-naphthalene, 2-thienyl, cyclopropyl and 2-, 3- and 4-pyridyl.

6. An unsaturated geminal phosphonate (III) according to claim 1 where R₃ is -H, sodium, potassium, calcium, magnesium, manganese, copper, gold, ethanolamine, diethanolamine, triethanolamine, zinc and THAM.

7. An unsaturated geminal phosphonate (III) according to claim 1 where R₃ is C₁-C₄ alkyl.

8. An unsaturated geminal phosphonate (III) according to claim 1 which is
[5-benzoyl-2,4-dihydro-3H-pyrazol-3-ylidene]bisphosphonic acid tetraethyl ester,
[5-(cyclohexylcarbonyl)-2,4-dihydro-3H-pyrazol-3-ylidene]bis-phosphonic acid tetraethyl ester,
[5-[2-(2-fluoro[1,1'-biphenyl]-4-yl)-1-oxopropyl]-2,4-dihydro-3H-pyrazol-3-ylidene]bisphosphonic acid tetraethyl ester,
[3-methyl-5(4H)-isoxazolylidene]bisphosphonic acid tetraethyl ester,
[3-phenyl-5(4H)-isoxazolylidene]bisphosphonic acid tetraethyl ester,
[2,4-dihydro-5-(1-oxopropyl)-3H-pyrazol-3-ylidene]bisphosphonic acid tetraethyl ester,
[2,4-dihydro-5-(4-nitrobenzoyl)-3H-pyrazol-3-ylidene]bisphosphonic acid tetraethyl ester,
[3-benzoyl-5(4H)-isoxazolylidene]bisphosphonic acid tetraethyl ester,
[5-(4'-chlorobenzoyl)-2,4-dihydro-3H-pyrazol-3-ylidene]bisphosphonic acid tetraethyl ester,
[5-(2',4'-dichlorobenzoyl)-2,4-dihydro-3H-pyrazol-3-ylidene]-bisphosphonic acid tetraethyl ester,
[5-(2,2-dimethyl-1-oxopropyl)-2,4-dihydro-3H-pyrazol-3-ylidene]-bisphosphonic acid tetraethyl ester,
[5-(2'-fluorobenzoyl)-2,4-dihydro-3H-pyrazol-3-ylidene]bisphosphonic acid tetraethyl ester,
[5-(4'-methoxybenzoyl)-2,4-dihydro-3H-pyrazol-3-ylidene]bisphosphonic acid tetraethyl ester,
[5-(4'-methylbenzoyl)-2,4-dihydro-3H-pyrazol-3-ylidene]bisphosphonic acid tetraethyl ester,
[5-(3'-methylbenzoyl)-2,4-dihydro-3H-pyrazol-3-ylidene]bisphosphonic acid tetraethyl ester,
[5-(3'-fluorobenzoyl)-2,4-dihydro-3H-pyrazol-3-ylidene]bisphosphonic acid tetraethyl ester,
[5-(2'-methylbenzoyl)-2,4-dihydro-3H-pyrazol-3-ylidene]bisphosphonic acid tetraethyl ester,
[5'-(4'-bromobenzoyl)-2,4-dihydro-3H-pyrazol-3-ylidene]-bisphosphonic acid tetraethyl ester,
[5'-(2',3',4'-trichlorobenzoyl)-2,4-dihydro-3H-pyrazol-3-ylidene]bisphosphonic acid tetraethyl ester,
[5'-(3'-methoxybenzoyl)-2,4-dihydro-3H-pyrazol-3-ylidene]-bisphosphonic acid tetraethyl ester,
[5'-(2',4'-dimethoxybenzoyl)-2,4-dihydro-3H-pyrazol-3-ylidene]-bisphosphonic acid tetraethyl ester,
[5'-(3'-trifluoromethylbenzoyl)-2,4-dihydro-3H-pyrazol-3-ylidene]bisphosphonic acid tetraethyl ester,
[5'-(cyclopropanoyl)-2,4-dihydro-3H-pyrazol-3-ylidene]bisphosphonic acid tetraethyl ester,
[5'-(9-'anthracenoyl)-2,4-dihydro-3H-pyrazol-3-ylidene]bisphosphonic acid tetraethyl ester,
[5'-(3',5'-difluorobenzoyl)-2,4-dihydro-3H-pyrazol-3-ylidene]-bisphosphonic acid tetraethyl ester.

9. An unsaturated geminal phosphonate (III) according to claim 1 which is
[5-benzoyl-2,4-dihydro-3H-pyrazol-3-ylidene]bisphosphonic acid tetraethyl ester,
[5-(cyclohexylcarbonyl-2,4-dihydro-3H-pyrazol-3-ylidene]bisphosphonic acid tetraethyl ester,
[5-[2-(2-fluoro[1,1'-biphenyl]-4-yl)-1-oxopropyl]-2,4-dihydro-3H-pyrazol-3-ylidene]bisphosphonic acid tetraethyl ester,
[3-methyl-5(4H)-isoxazolylidene]bisphosphonic acid tetraethyl ester,
[3-phenyl-5(4H)-isoxazolylidene]bisphosphonic acid tetraethyl ester,
[2,4-dihydro-5-(4-nitrobenzoyl)-3H-pyrazol-3-ylidene]bisphosphonic acid tetraethyl ester,
[3-benzoyl-5(4H)-isoxazolylidene]bisphosphonic acid tetraethyl ester,
[5-(4'-chlorobenzoyl)-2,4-dihydro-3H-pyrazol-3-ylidene]bisphosphonic acid tetraethyl ester,
[5-(2',4'-dichlorobenzoyl)-2,4-dihydro-3H-pyrazol-3-ylidene]bisphosphonic acid tetraethyl ester,
[5-(2',2'-dimethyl-1-oxopropyl)-2,4-dihydro-3H-pyrazol-3-ylidene]bisphosphonic acid tetraethyl ester,
[5-(2'-fluorobenzoyl)-2,4-dihydro-3H-pyrazol-3-ylidene]bisphosphonic acid tetraethyl ester,
[5-(4'-methoxybenzoyl)-2,4-dihydro-3H-pyrazol-3-ylidene]bisphosphonic acid tetraethyl ester,
[5-(4'-methylbenzoyl)-2,4-dihydro-3H-pyrazol-3-ylidene]bisphosphonic acid tetraethyl ester,
[5-(3'-methylbenzoyl)-2,4-dihydro-3H-pyrazol-3-ylidene]bisphosphonic acid tetraethyl ester,
[5-(3'-fluorobenzoyl)-2,4-dihydro-3H-pyrazol-3-ylidene]bisphosphonic acid tetraethyl ester,
[5-(2'-methylbenzoyl)-2,4-dihydro-3H-pyrazol-3-ylidene]bisphosphonic acid tetraethyl ester,
[5'-(4'-bromobenzoyl)-2,4-dihydro-3H-pyrazol-3-ylidene]-bisphosphonic acid tetraethyl ester,
[5'-(2',3',4'-trichlorobenzoyl)-2,4-dihydro-3H-pyrazol-3-ylidene]bisphosphonic acid tetraethyl ester,
[5'-(3'-methoxybenzoyl)-2,4-dihydro-3H-pyrazol-3-ylidene]bisphosphonic acid tetraethyl ester,
[5'-(2',4'-dimethoxybenzoyl)-2,4-dihydro-3H-pyrazol-3-ylidene]bisphosphonic acid tetraethyl ester,
[5'-(3'-trifluoromethylbenzoyl)-2,4-dihydro-3H-pyrazol-3-ylidene]bisphosphonic acid tetraethyl ester,
[5'-(cyclopropanoyl)-2,4-dihydro-3H-pyrazol-3-ylidene]bisphosphonic acid tetraethyl ester,
[5'-(9-'anthracenoyl)-2,4-dihydro-3H-pyrazol-3-ylidene]bisphosphonic acid tetraethyl ester,
[5'-(3',5'-difluorobenzoyl)-2,4-dihydro-3H-pyrazol-3-ylidene]bisphosphonic acid tetraethyl ester.

10. An unsaturated geminal phosphonate (III) according to claim 1 which is
[5-(2'-bromobenzoyl)-2,4-dihydro-3H-pyrazol-3-ylidene]bisphosphonic acid tetraethyl ester,
[5-(3'-bromobenzoyl)-2,4-dihydro-3H-pyrazol-3-ylidene]bisphosphonic acid tetraethyl ester,
[5-(2'-chlorobenzoyl)-2,4-dihydro-3H-pyrazol-3-ylidene]bisphosphonic acid tetraethyl ester,
[5-(3'-chlorobenzoyl)-2,4-dihydro-3H-pyrazol-3-ylidene]bisphosphonic acid tetraethyl ester,
[5-(3',4'-dichlorobenzoyl)-2,4-dihydro-3H-pyrazol-3-ylidene]bisphosphonic acid tetraethyl ester,
[5-(3',5'-dichlorobenzoyl)-2,4-dihydro-3H-pyrazol-3-ylidene]bisphosphonic acid tetraethyl ester,
[5-(2',6'-dichlorobenzoyl)-2,4-dihydro-3H-pyrazol-3-ylidene]bisphosphonic acid tetraethyl ester,
[5-(4'-fluorobenzoyl)-2,4-dihydro-3H-pyrazol-3-ylidene]bisphosphonic acid tetraethyl ester,
[5-(2',3',4',5',6''-pentafluorobenzoyl)-2,4-dihydro-3H-pyrazol-3-ylidene]bisphosphonic acid tetraethyl ester,
[5-(2'-methoxybenzoyl)-2,4-dihydro-3H-pyrazol-3-ylidene]bisphosphonic acid tetraethyl ester,
[5-(3',5'-dimethoxybenzoyl)-2,4-dihydro-3H-pyrazol-3-ylidene]bisphosphonic acid tetraethyl ester,
[5-(2'-chloro-4'-methoxybenzoyl)-2,4-dihydro-3H-pyrazol-3-ylidene]bisphosphonic acid tetraethyl ester,
[5-(4'-chloro-2'-methoxybenzoyl)-2,4-dihydro-3H-pyrazol-3-ylidene]bisphosphonic acid tetraethyl ester,
[5-(3'-chloro-4'-methoxybenzoyl)-2,4-dihydro-3H-pyrazol-3-ylidene]bisphosphonic acid tetraethyl ester,
[5-(4'-chloro-3'-methoxybenzoyl)-2,4-dihydro-3H-pyrazol-3-ylidene]bisphosphonic acid tetraethyl ester,
[5-(3'-methoxybenzoyl)-2,4-dihydro-3H-pyrazol-3-ylidene]bisphosphonic acid tetraethyl ester,
[5-(2'-ethoxybenzoyl)-2,4-dihydro-3H-pyrazol-3-ylidene]bisphosphonic acid tetraethyl ester,
[5-(3'-ethoxybenzoyl)-2,4-dihydro-3H-pyrazol-3-ylidene]bisphosphonic acid tetraethyl ester,
[5-(4'-ethoxybenzoyl)-2,4-dihydro-3H-pyrazol-3-ylidene]bisphosphonic acid tetraethyl ester,
[5-(2'-phenoxybenzoyl)-2,4-dihydro-3H-pyrazol-3-ylidene]bisphosphonic acid tetraethyl ester,
[5-(3'-phenoxybenzoyl)-2,4-dihydro-3H-pyrazol-3-ylidene]bisphosphonic acid tetraethyl ester,
[5-(4'-phenoxybenzoyl)-2,4-dihydro-3H-pyrazol-3-ylidene]bisphosphonic acid tetraethyl ester,
[5-(2'-methylthiobenzoyl)-2,4-dihydro-3H-pyrazol-3-ylidene]bisphosphonic acid tetraethyl ester,
[5-(3'-methylthiobenzoyl)-2,4-dihydro-3H-pyrazol-3-ylidene]bisphosphonic acid tetraethyl ester,
[5-(4'-methylthiobenzoyl)-2,4-dihydro-3H-pyrazol-3-ylidene]bisphosphonic acid tetraethyl ester,
[5-(2'-chloro-4'-methylbenzoyl)-2,4-dihydro-3H-pyrazol-3-ylidene]bisphosphonic acid tetraethyl ester,
[5-(4'-chloro-2'-methylbenzoyl)-2,4-dihydro-3H-pyrazol-3-ylidene]bisphosphonic acid tetraethyl ester,
[5-(3'-chloro-4'-methylbenzoyl)-2,4-dihydro-3H-pyrazol-3-ylidene]bisphosphonic acid tetraethyl ester,
[5-(4'-chloro-3'-methylbenzoyl)-2,4-dihydro-3H-pyrazol-3-ylidene]bisphosphonic acid tetraethyl ester,
[5-(2'-ethylbenzoyl)-2,4-dihydro-3H-pyrazol-3-ylidene]bisphosphonic acid tetraethyl ester,
[5-(3'-ethylbenzoyl)-2,4-dihydro-3H-pyrazol-3-ylidene]bisphosphonic acid tetraethyl ester,
[5-(4'-ethylbenzoyl)-2,4-dihydro-3H-pyrazol-3-ylidene]bisphosphonic acid tetraethyl ester,
[5-(4'-tert-butylbenzoyl)-2,4-dihydro-3H-pyrazol-3-ylidene]bisphosphonic acid tetraethyl ester,
[5-(2',4'-dimethylbenzoyl)-2,4-dihydro-3H-pyrazol-3-ylidene]bisphosphonic acid tetraethyl ester,
[5-(3',5'-dimethylbenzoyl)-2,4-dihydro-3H-pyrazol-3-ylidene]bisphosphonic acid tetraethyl ester,
[5-(2'-phenylbenzoyl)-2,4-dihydro-3H-pyrazol-3-ylidene]bisphosphonic acid tetraethyl ester,
[5-(3'-phenylbenzoyl)-2,4-dihydro-3H-pyrazol-3-ylidene]bisphosphonic acid tetraethyl ester,
[5-(4'-phenylbenzoyl)-2,4-dihydro-3H-pyrazol-3-ylidene]bisphosphonic acid tetraethyl ester,
[5-(4'-morpholinobenzoyl)-2,4-dihydro-3H-pyrazol-3-ylidene]bisphosphonic acid tetraethyl ester,
[5-(4'-dimethylaminobenzoyl)-2,4-dihydro-3H-pyrazol-3-ylidene]bisphosphonic acid tetraethyl ester,
[5-(4'-diethylaminobenzoyl)-2,4-dihydro-3H-pyrazol-3-ylidene]bisphosphonic acid tetraethyl ester,
[5-(1'-naphthoyl)-2,4-dihydro-3H-pyrazol-3-ylidene)bisphosphonic acid tetraethyl ester,
[5-(2'-naphthoyl)-2,4-dihydro-3H-pyrazol-3-ylidene]bisphosphonic acid tetraethyl ester,
[5-(6'-quinolinoyl)-2,4-dihydro-3H-pyrazol-3-ylidene]bisphosphonic acid tetraethyl ester,
[5-(8'-quinolinoyl)-2,4-dihydro-3H-pyrazol-3-ylidene]bisphosphonic acid tetraethyl ester,
[5-(2'-thienylcarbonyl)-2,4-dihydro-3H-pyrazol-3-ylidene]bisphosphonic acid tetraethyl ester,
[5-(2'-pyridylcarbonyl)-2,4-dihydro-3H-pyrazol-3-ylidene]bisphosphonic acid tetraethyl ester,
[5-(nicotinoyl)-2,4-dihydro-3H-pyrazol-3-ylidene]bisphosphonic acid tetraethyl ester,
[5-(4'-pyridylcarbonyl)-2,4-dihydro-3H-pyrazol-3-ylidene]bisphosphonic acid tetraethyl ester,
[5-(cyclobutanoyl)-2,4-dihydro-3H-pyrazol-3-ylidene)bisphosphonic acid tetraethyl ester and
[5-(cyclopentanoyl)-2,4-dihydro-3H-pyrazol-3-ylidene)bisphosphonic acid tetraethyl ester.

11. [5-(3'-Fluorobenzoyl)-2,4-dihydro-3H-pyrazol-3-ylidene)-bisphosphonic acid tetraethyl ester.

12. [5'-(2',3',4'-Trichlorobenzoyl)-2,4-dihydro-3H-pyrazol-3-ylidene)bisphosphonic acid tetraethyl ester.

## Patentansprüche

1. Ungesättigtes gem-Phosphonat der Formel (III) 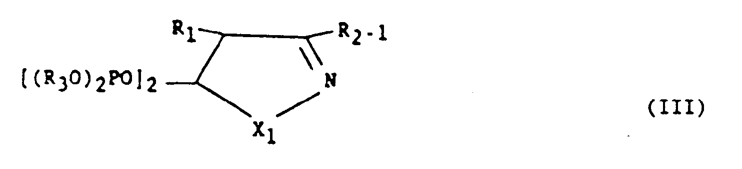 worin bedeuten:
X₁ -O-, -NH- oder -N-Metall, wobei das Metall aus Natrium, Kalium, Calcium, Magnesium, Kupfer, Zink, Barium, Silber und Gold besteht;
R₁ H, C₁-C₄ Alkyl, C₃-C₇ Cycloalkyl, -φ gegebenenfalls substituiert mit 1 bis 5 -F, -Cl, -Br, -I, -CF₃, C₁-C₆ Alkyl, C₁-C₆ Cycloalkyl, C₁-C₄ Alkoxy oder
C₁-C₄ Alkylthio;
R₂₋₁ C₁-C₆ Alkyl, C₃-C₇ Cycloalkyl, -φ gegebenenfalls substituiert mit 1 bis 5 -F, -Cl, -Br, -I, -NO₂, -CN, -CF₃, C₁-C₆ Alkyl, C₃-C₇ Cycloalkyl, C₁-C₄ Alkoxy, C₁-C₄ Alkylthio,
-CH(OH)-R₂₋₅ mit R₂₋₅ gleich
(A) C₁-C₁₀ Alkyl,
(B) C₃-C₇ Cycloalkyl,
(C) -φ gegebenenfalls substituiert mit 1 oder 2 -φ oder 1 bis 5 -F, -Cl, -Br, -I, -NO₂, -CN, -CF₃, C₁-C₆ Alkyl, C₃-C₇ Cycloalkyl, C₁-C₄ Alkoxy, C₁-C₄ Alkylthio,
(D) 2- und 3-Furanyl, gegebenenfalls substituiert mit 1 bis 3 -F, Cl, Br, -I, -NO₂, -CN, -CF₃, C₁-C₆ Alkyl, C₃-C₇ Cycloalkyl, C₁-C₄ Alkoxy, -O-φ, C₁-C₄ Alkylthio,
(E) 2-, 4- und 5-Pyrimidyl, gegebenenfalls substituiert mit 1 bis 3 -F, -Cl, -Br, -I, -NO₂, -CN, -CF₃, C₁-C₆ Alkyl, C₃-C₇ Cycloalkyl, C₁-C₄ Alkoxy, C₁-C₄ Alkylthio,
(F) 2-, 3- und 4-Pyridinyl, gegebenenfalls substituiert mit 1 bis 4 -F, -Cl, -Br, -I, NO₂, -CN, -CF₃, C₁-C₆ Alkyl, C₃-C₇ Cycloalkyl, C₁-C₄ Alkoxy C₁-C₄ Alkylthio,
(G) 2- und 3-Thiophen, gegebenenfalls substituiert mit 1 bis 3 -F, -Cl, -Br, -I, -NO₂, -CN, -CF₃, C₁-C₆ Alkyl, C₃-C₇ Cycloalkyl, C₁-C₄ Alkoxy, C₁-C₄ Alkylthio,
(H) 1- und 2-Naphthalin, gegebenenfalls substituiert mit 1 bis 7 -F, -Cl, -Br, -I, -NO₂, -CN, -CF₃, C₁-C₆ Alkyl, C₃-C₇ Cycloalkyl, C₁-C₄ Alkoxy, C₁-C₄ Alkylthio,
(I) 2- 3-, 4-, 6-, 7- und 8-Chinolin
(J) 1-, 3-, 4-, 6-, 7- und 8-Isochinolin,
(K) 2-, 3-, 4-, 5-, 6- und 7-Benzothiophen,
(L) 2-, 3-, 4-, 6- und 7-Benzofuran,
(M) -NR₂₋₆R₂₋₇, wobei R₂₋₆ und R₂₋₇ gleich oder verschieden sind und für C₁-C₄ Alkyl, -φ, -CO-R₂₋₈ mit R₂₋₈ gleich C₁-C₄ Alkyl oder -φ gegebenenfalls substituiert mit 1 -CH₃, -SO₂-R₂₋₈ mit R₂₋₈ in der angegeben Definition und worin R₂₋₆ und R₂₋₇ zusammen mit dem daran hängenden Stickstoffatom einen 4- bis 8-gliedrigen heterocyclischen Ring mit einem Stickstoff, Sauerstoff- oder Schwefelheteroatom und 0 bis 3 Doppelbindungen bilden können,
-CO-R₂₋₅ mit R₂₋₅ in der angegebenen Bedeutung;
R₃ -H, C₁-C₆ Alkyl, -φ und deren pharmazeutisch akzeptablen Salze.

2. Ungesättigtes gem-Phosphonat (III) nach Anspruch 1 mit X₁ gleich -NH-.

3. Ungesättigtes gem-Phosphonat (III) nach Anspruch 1 mit R₁ gleich -H.

4. Ungesättigtes gem-Phosphonat (III) nach Anspruch 1 mit R₂₋₁ gleich -CO-R₂₋₅ und -CH(OH)-R₂₋₅.

5. Ungesättigtes gem-Phosphonat (III) nach Anspruch 1 mit R₂₋₁ gleich- -CO-φ gegebenenfalls substituiert mit -F, -Cl, -Br, -I, -CN, -CF₃, C₁-C₄ Alkoxy, C₁-C₄ Alkyl, -N(CH₃)₂, -N(CH₂CH₃)₂, Morpholino, 1- und 2-Naphthalin, 2-Thienyl, Cyclopropyl und 2-, 3- und 4-Pyridyl.

6. Ungesättigtes gem-Phosphonat (III) nach Anspruch 1 mit R₃ gleich -H, Natrium, Kalium, Calcium, Magnesium, Mangan, Kupfer, Gold, Ethanolamin, Diethanolamin, Triethanolamin, Zink und THAM.

7. Ungesättigtes gem-Phosphonat (III) nach Anspruch 1 mit R₃ gleich C₁-C₄ Alkyl.

8. Ungesättigtes gem-Phosphonat (III) nach Anspruch 1, nämlich
[5-Benzoyl-2,4-dihydro-3H-pyrazol-3-yliden]-bisphosphonsäure-tetraethylester,
[5-(Cyclohexylcarbonyl)-2,4-dihydro-3H-pyrazol-3-yliden]-bisphosphonsäure-tetraethylester,
[5-[2-(2-Fluor-[1,1'-biphenyl]-4-yl)-1-oxopropyl]-2,4-dihydro-3H-pyrazol-3-yliden]-bisphosphonsäuretetraethylester,
[3-Methyl-5(4H)-isoxazolyliden]-bisphosphonsäuretetraethylester,
[3-Phenyl-5(4H)-isoxazolyliden]-bisphosphonsäuretetraethylester,
[2,4-Dihydro-5-(1-oxopropyl)-3H-pyrazol-3-yliden]-bisphosphonsäure-tetraethylester,
[2,4-Dihydro-5-(4-nitrobenzoyl)-3H-pyrazol-3-yliden]-bisphosphonsäure-tetraethylester,
[3-Benzoyl-5(4H)-isoxazolyliden]-bisphosphonsäuretetraethylester,
[5-(4'-Chlorbenzoyl)-2,4-dihydro-3H-pyrazol-3-yliden]-bisphosphonsäure-tetraethylester,
[5-(2',4'-Dichlorbenzoyl)-2,4-dihydro-3H-pyrazol-3-yliden]-bisphosphonsäure-tetraethylester,
[5-(2,2-Dimethyl-1-oxopropyl)-2,4-dihydro-3H-pyrazol-3-yliden]-bisphosphonsäure-tetraethylester,
[5-(2'-Fluorbenzoyl)-2,4-dihydro-3H-pyrazol-3-yliden]-bisphosphonsäure-tetraethylester,
[5-(4'-Methoxyberizoyl)-2,4-dihydro-3H-pyrazol-3-yliden]-bisphosphonsäure-tetraethylester,
[5-(4'-Methylbenzoyl)-2,4-dihydro-3H-pyrazol-3-yliden]-bisphosphonsäure-tetraethylester,
[5-(3'-Methylbenzoyl)-2,4-dihydro-3H-pyrazol-3-yliden]-bisphosphonsäure-tetraethylester,
[5-(3'-Fluorbenzoyl)-2,4-dihydro-3H-pyrazol-3-yliden]-bisphosphonsäure-tetraethylester,
[5-(2'-Methylbenzoyl)-2,4-dihydro-3H-pyrazol-3-yliden]-bisphosphonsäure-tetraethylester,
[5'-(4'-Brombenzoyl)-2,4-dihydro-3H-pyrazol-3-yliden]-bisphosphonsäure-tetraethylester,
[5'-(2',3',4'-Trichlorbenzoyl)-2,4-dihydro-3H-pyrazol-3-yliden]-bisphosphonsäure-tetraethylester,
[5'-(3'-Methoxybenzoyl)-2,4-dihydro-3H-pyrazol-3-yliden]-bisphosphonsäure-tetraethylester,
[5'-(2',4'-Dimethoxybenzoyl)-2,4-dihydro-3H-pyrazol-3-yliden]-bisphosphonsäure-tetraethylester,
[5'-(3'-Trifluormethylbenzoyl)-2,4-dihydro-3H-pyrazol-3-yliden]-bisphosphonsäure-tetraethylester,
[5'-(Cyclopropanoyl)-2,4-dihydro-3H-pyrazol-3-yliden]-bisphosphonsäure-tetraethylester,
[5'-(9'-Anthracenoyl)-2,4-dihydro-3H-pyrazol-3-yliden]-bisphosphonsäure-tetraethylester,
[5'-(3',5'-Difluorbenzoyl)-2,4-dihydro-3H-pyrazol-3-yliden]-bisphosphonsäure-tetraethylester.

9. Ungesättigtes gem-Phosphonat (III) nach Anspruch 1, nämlich
[5-Benzoyl-2,4-dihydro-3H-pyrazol-3-yliden]-bisphosphonsäure-tetraethylester,
[5-(Cyclohexylcarbonyl-2,4-dihydro-3H-pyrazol-3-yliden]-bisphosphonsäure-tetraethylester,
[5-[2-(2-Fluor-[1,1'-biphenyl]-4-yl)-1-oxopropyl]-2,4-dihydro-3H-pyrazol-3-yliden]-bisphosphonsäuretetraethylester,
[3-Methyl-5(4H)-isoxazolyliden]-bisphosphonsäuretetraethylester,
[3-Phenyl-5(4H)-isoxazolyliden]-bisphosphonsäuretetraethylester,
[2,4-Dihydro-5-(4-nitrobenzoyl)-3H-pyrazol-3-yliden]-bisphosphonsäure-tetraethylester,
[3-Benzoyl-5(4H)-isoxazolyliden]-bisphosphonsäuretetraethylester,
[5-(4'-Chlorbenzoyl)-2,4-dihydro-3H-pyrazol-3-yliden]-bisphosphonsäure-tetraethylester,
[5-(2',4'-Dichlorbenzoyl)-2,4-dihydro-3H-pyrazol-3-yliden]-bisphosphonsäure-tetraethylester,
[5-(2',2'-Dimethyl-1-oxopropyl)-2,4-dihydro-3H-pyrazol-3-yliden]-bisphosphonsäure-tetraethylester,
[5-(2'-Fluorbenzoyl)-2,4-dihydro-3H-pyrazol-3-yliden]-bisphosphonsäure-tetraethylester,
5-(4'-Methoxybenzoyl)-2,4-dihydro-3H-pyrazol-3-yliden]-bisphosphonsäure-tetraethylester,
[5-(4'-Methylbenzoyl)-2,4-dihydro-3H-pyrazol-3-yliden]-bisphosphonsäure-tetraethylester,
[5-(3'-Methylbenzoyl)-2,4-dihydro-3H-pyrazol-3-yliden]-bisphosphonsäure-tetraethylester,
[5-(3'-Fluorbenzoyl)-2,4-dihydro-3H-pyrazol-3-yliden]-bisphosphonsäure-tetraethylester,
[5-(2'-Methylbenzoyl)-2,4-dihydro-3H-pyrazol-3-yliden]-bisphosphonsäure-tetraethylester,
[5'-(4'-Brombenzoyl)-2,4-dihydro-3H-pyrazol-3-yliden]-bisphosphonsäure-tetraethylester,
[5'-(2',3',4'-Trichlorbenzoyl)-2,4'-dihydro-3H-pyrazol-3-yliden]-bisphosphonsäure-tetraethylester,
[5'-(3'-Methoxybenzoyl)-2,4-dihydro-3H-pyrazol-3-yliden]-bisphosphonsäure-tetraethylester,
[5'-(2',4'-Dimethoxybenzoyl)-2,4-dihydro-3H-pyrazol-3-yliden]-bisphosphonsäure-tetraethylester,
[5'-(3'-Trifluormethylbenzoyl)-2,4-dihydro-3H-pyrazol-3-yliden]-bisphosphonsäure-tetraethylester,
[5'-(Cyclopropanoyl)-2,4-dihydro-3H-pyrazol-3-yliden]-bisphosphonsäure-tetraethylester,
[5'-(9'-Anthracenoyl)-2,4-dihydro-3H-pyrazol-3-yliden]-bisphosphonsäure-tetraethylester oder
[5'-(3',5'-Difluorbenzoyl)-2,4-dihydro-3H-pyrazol-3-yliden]-bisphosphonsäure-tetraethylester.

10. Ungesättigtes gem-Phosphonat (III) nach Anspruch 1, nämlich
[5-(2'-Brombenzoyl)-2,4-dihydro-3H-pyrazol-3-yliden]-bisphosphonsäure-tetraethylester,
[5-(3'-Brombenzoyl)-2,4-dihydro-3H-pyrazol-3-yliden]-bisphosphonsäure-tetraethylester,
[5-(2'-Chlorbenzoyl)-2,4-dihydro-3H-pyrazol-3-yliden]-bisphosphonsäure-tetraethylester,
[5-(3'-Chlorbenzoyl)-2,4-dihydro-3H-pyrazol-3-yliden]-bisphosphonsäure-tetraethylester,
[5-(3',4'-Dichlorbenzoyl)-2,4-dihydro-3H-pyrazol-3-yliden]-bisphosphonsäure-tetraethylester,
[5-(3',5'-Dichlorbenzoyl)-2,4-dihydro-3H-pyrazol-3-yliden]-bisphosphonsäure-tetraethylester,
[5-(2',6'-Dichlorbenzoyl)-2,4-dihydro-3H-pyrazol-3-yliden]-bisphosphonsäure-tetraethylester,
[5-(4'-Fluorbenzoyl)-2,4-dihydro-3H-pyrazol-3-yliden]-bisphosphonsäure-tetraethylester,
[5-(2',3',4',5',6''-Pentafluorbenzoyl)-2,4-dihydro-3H-pyrazol-3-yliden]-bisphosphonsäure-tetraethylester,
[5-(2'-Methoxybenzoyl)-2,4-dihydro-3H-pyrazol-3-yliden]-bisphosphonsäure-tetraethylester,
[5-(3',5'-Dimethoxybenzoyl)-2,4-dihydro-3H-pyrazol-3-yliden]-bisphosphonsäure-tetraethylester,
[5-(2'-Chlor-4'-methoxybenzoyl)-2,4-dihydro-3H-pyrazol-3-yliden]-bisphosphonsäure-tetraethylester,
[5-(4'-Chlor-2'-methoxybenzoyl)-2,4-dihydro-3H-pyrazol-3-yliden]-bisphosphonsäure-tetraethylester,
[5-(3'-Chlor-4'-methoxybenzoyl)-2,4-dihydro-3H-pyrazol-3-yliden]-bisphosphonsäure-tetraethylester,
[5-(4'-Chlor-3'-methoxybenzoyl)-2,4-dihydro-3H-pyrazol-3-yliden]-bisphosphonsäure-tetraethylester,
[5-(3'-Methoxybenzoyl)-2,4-dihydro-3H-pyrazol-3-yliden]-bisphosphonsäure-tetraethylester,
[5-(2'-Ethoxybenzoyl)-2,4-dihydro-3H-pyrazol-3-yliden]-bisphosphonsäure-tetraethylester,
[5-(3'-Ethoxybenzoyl)-2,4-dihydro-3H-pyrazol-3-yliden]-bisphosphonsäure-tetraethylester,
[5-(4'-Ethoxybenzoyl)-2,4-dihydro-3H-pyrazol-3-yliden]-bisphosphonsäure-tetraethylester,
[5-(2'-Phenoxybenzoyl)-2,4-dihydro-3H-pyrazol-3-yliden]-bisphosphonsäure-tetraethylester,
[5-(3'-Phenoxybenzoyl)-2,4-dihydro-3H-pyrazol-3-yliden]-bisphosphonsäure-tetraethylester,
[5-(4'-Phenoxybenzoyl)-2,4-dihydro-3H-pyrazol-3-yliden]-bisphosphonsäure-tetraethylester,
[5-(2'-Methylthiobenzoyl)-2,4-dihydro-3H-pyrazol-3-yliden]-bisphosphonsäure-tetraethylester,
[5-(3'-Methylthiobenzoyl)-2,4-dihydro-3H-pyrazol-3-yliden]-bisphosphonsäure-tetraethylester,
[5-(4'-Methylthiobenzoyl)-2,4-dihydro-3H-pyrazol-3-yliden]-bisphosphonsäure-tetraethylester,
[5-(2'-Chlor-4'-methylbenzoyl)-2,4-dihydro-3H-pyrazol-3-yliden]-bisphosphonsäure-tetraethylester,
[5-(4'-Chlor-2'-methylbenzoyl)-2,4-dihydro-3H-pyrazol-3-yliden]-bisphosphonsäure-tetraethylester,
[5-(3'-Chlor-4'-methylbenzoyl)-2,4-dihydro-3H-pyrazol-3-yliden]-bisphosphonsäure-tetraethylester,
[5-(4'-Chlor-3'-methylbenzoyl)-2,4-dihydro-3H-pyrazol-3-yliden]-bisphosphonsäure-tetraethylester,
[5-(2'-Ethylbenzoyl)-2,4-dihydro-3H-pyrazol-3-yliden]-bisphosphonsäure-tetraethylester,
[5-(3'-Ethylbenzoyl)-2,4-dihydro-3H-pyrazol-3-yliden]-bisphosphonsäure-tetraethylester,
[5-(4'-Ethylbenzoyl)-2,4-dihydro-3H-pyrazol-3-yliden]-bisphosphonsäure-tetraethylester,
[5-(4'-tert.-Butylbenzoyl)-2,4-dihydro-3H-pyrazol-3-yliden]-bisphosphonsäure-tetraethylester,
[5-(2',4'-Dimethylbenzoyl)-2,4-dihydro-3H-pyrazol-3-yliden]-bisphosphonsäure-tetraethylester,
[5-(3',5'-Dimethylbenzoyl)-2,4-dihydro-3H-pyrazol-3-yliden]-bisphosphonsäure-tetraethylester,
[5-(2'-Phenylbenzoyl)-2,4-dihydro-3H-pyrazol-3-yliden]-bisphosphonsäure-tetraethylester,
[5-(3'-Phenylbenzoyl)-2,4-dihydro-3H-pyrazol-3-yliden]-bisphosphonsäure-tetraethylester,
[5-(4'-Phenylbenzoyl)-2,4-dihydro-3H-pyrazol-3-yliden]-bisphosphonsäure-tetraethylester,
[5-(4'-Morpholinobenzoyl)-2,4-dihydro-3H-pyrazol-3-yliden]-bisphosphonsäure-tetraethylester,
[5-(4'-Dimethylaminobenzoyl)-2,4-dihydro-3H-pyrazol-3-yliden]-bisphosphonsäure-tetraethylester,
[5-(4'-Diethylaminobenzoyl)-2,4-dihydro-3H-pyrazol-3-yliden]-bisphosphonsäure-tetraethylester,
[5-(1'-Naphthoyl)-2,4-dihydro-3H-pyrazol-3-yliden]-bisphosphonsäure-tetraethylester,
[5-(2'-Naphthoyl)-2,4-dihydro-3H-pyrazol-3-yliden]-bisphosphonsäure-tetraethylester,
[5-(6'-Chinolinoyl)-2,4-dihydro-3H-pyrazol-3-yliden]-bisphosphonsäure-tetraethylester,
[5-(8'-Chinolinoyl)-2,4-dihydro-3H-pyrazol-3-yliden]-bisphosphonsäure-tetraethylester,
[5-(2'-Thienylcarbonyl)-2,4-dihydro-3H-pyrazol-3-yliden]-bisphosphonsäure-tetraethylester,
[5-(2'-Pyridylcarbonyl)-2,4-dihydro-3H-pyrazol-3-yliden]-bisphosphonsäure-tetraethylester,
[5-(Nicotinoyl)-2,4-dihydro-3H-pyrazol-3-yliden]-bisphosphonsäure-tetraethylester,
[5-(4'-Pyridylcarbonyl)-2,4-dihydro-3H-pyrazol-3-yliden]-bisphosphonsäure-tetraethylester,
[5-(Cyclobutanoyl)-2,4-dihydro-3H-pyrazol-3-yliden]-bisphosphonsäure-tetraethylester oder
[5-(Cyclopentanoyl)-2,4-dihydro-3H-pyrazol-3-yliden]-bisphosphonsäure-tetraethylester.

11. [5-(3'-Fluorbenzoyl)-2,4-dihydro-3H-pyrazol-3-yliden]-bisphosphonsäure-tetraethylester.

12. [5'-(2',3',4'-Trichlorbenzoyl)-2,4-dihydro-3H-pyrazol-3-yliden]-bisphosphonsäure-tetraethylester.

## Revendications

1. Phosphonate géminé insaturé de formule (III) 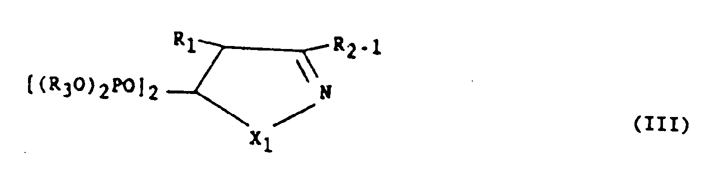 dans laquelle
X₁ représente un groupe -O-, -NH- ou -N-métal dans lequel le métal est choisi entre le sodium, le potassium, le calcium, le magnésium, le cuivre, le zinc, le baryum, l'argent et l'or ;
R₁ représente -H, un groupe alkyle en C₁ à C₄, cycloalkyle en C₃ à C₇, -φ portant facultativement 1 à 5 substituants -F, -Cl, -Br, -I, -CF₃, alkyle en C₁ à C₆, cycloalkyle en C₁ à C₆, alkoxy en C₁ à C₄ ou alkylthio en C₁ à C₄ ;
R₂₋₁ représente un groupe
alkyle en C₁ à C₆,
cycloalkyle en C₃ à C₇,
-φ portant facultativement 1 à 5 substituants -F, -CI, -Br, -I, -NO₂, -CN, -CF₃, alkyle en C₁ à C₆, cycloalkyle en C₃ à C₇, alkoxy en C₁ à C₄, alkylthio en C₁ à C₄,
-CH(OH)-R₂₋₅ dans lequel R₂₋₅ représente un groupe
(A) alkyle en C₁ à C₁₀,
(B) cycloalkyle en C₃ à C₇,
(C) -φ portant facultativement 1 ou 2 substituants -φ ou 1 à 5 substituants -F, -Cl, -Br, -I, -NO₂, -CN, -CF₃, alkyle en C₁ à C₆, cycloalkyle en C₃ à C₇, alkoxy en C₁ à C₄, alkylthio en C₁ à C₄,
(D) 2- ou 3-furannyle portant facultativement 1 à 3 substituants -F, -Cl, -Br, -I, -NO₂, -CN, -CF₃, alkyle en C₁ à C₆, cycloalkyle en C₃ à C₇, alkoxy en C₁ à C₄, -O-φ, alkylthio en C₁ à C₄,
(E) 2-, 4- ou 5-pyrimidyle portant facultativement 1 à 3 substituants -F, -Cl, -Br, -I, -NO₂, -CN, -CF₃, alkyle en C₁ à C₆, cycloalkyle en C₃ à C₇, alkoxy en C₁ à C₄, alkylthio en C₁ à C₄,
(F) 2-, 3- ou 4-pyridinyle portant facultativement 1 à 4 substituants -F, -Cl, -Br, -I, -NO₂, -CN, -CF₃, alkyle en C₁ à C₆, cycloalkyle en C₃ à C₇, alkoxy en C₁ à C₄, alkylthio en C₁ à C₄,
(G) 2- ou 3-thiophène portant facultativement 1 à 3 substituants -F, -Cl, -Br, -I, -NO₂, -CN, -CF₃, alkyle en C₁ à C₆, cycloalkyle en C₃ à C₇, alkoxy en C₁ à C₄, alkylthio en C₁ à C₄,
(H) 1- ou 2-naphtalène portant facultativement 1 à 7 substituants -F, Cl, -BR, -I, -NO₂, -CN, -CF₃, alkyle en C₁ à C₆, cycloalkyle en C₃ à C₇, alkoxy en C₁ à C₄, alkylthio en C₁ à C₄,
(I) 2-, 3-, 4-, 6-, 7- ou 8-quinoléine,
(J) 1-, 3-, 4-, 6-, 7- ou 8-isoquinoléine,
(K) 2-, 3-, 4-, 5-, 6- ou 7-benzothiophène,
(L) 2-, 3-, 4-, 5-, 6- ou 7-benzofuranne,
(M) -NR₂₋₆R₂₋₇ dans lequel R₂₋₆ et R₂₋₇ sont identiques ou différents et représentent des groupes alkyle en C₁ à C₄,
-φ,
-CO-R₂₋₈ dans lequel R₂₋₈ représente un groupe alkyle en C₁ à C₄ ou -φ facultativement substitué avec un groupe -CH₃,
-SO₂R₂₋₈ dans lequel R₂₋₈ répond à la définition précitée et R₂₋₆ et R₂₋₇, pris conjointement avec l'atome d'azote auquel ils sont fixés, forment un noyau hétérocyclique tétra à octogonal contenant un hétéro-atome consistant en azote, oxygène ou soufre et 0 à 3 doubles liaisons,
-CO-R₂₋₅ dans lequel R₂₋₅ répond à la définition précitée ;
R₃ représente -H, un groupe alkyle en C₁ à C₆, -φ, et ses sels pharmaceutiquement acceptables.

2. Phosphate géminé insaturé de formule (III) suivant la revendication 1, dans lequel X₁ représente un groupe -NH-.

3. Phosphate géminé insaturé de formule (III) suivant la revendication 1, dans lequel R₁ représente -H.

4. Phosphate géminé insaturé de formule (III) suivant la revendication 1, dans lequel R₂₋₁ est choisi entre des groupes -CO-R₂₋₅ et -CH(OH)-R₂₋₅.

5. Phosphate géminé insaturé de formule (III) suivant la revendication 1, dans lequel R₂₋₁ représente un groupe -CO-φ facultativement substitué avec des groupes -F, -Cl, -Br, -I, -CN, -CF₃, alkoxy en C₁ à C₄, alkyle en C₁ à C₄, -N(CH₃)₂, -N(CH₂CH₃)₂, morpholino, 1- et 2-naphtalène, 2-thiényle, cyclopropyle ou 2-, 3- et 4-pyridyle.

6. Phosphate géminé insaturé de formule (III) suivant la revendication 1, dans lequel R₃ représente -H, le sodium, le potassium, le calcium, le magnésium, le manganèse, le cuivre, l'or, l'éthanolamine, la diéthanolamine, la triéthanolamine, le zinc ou le THAM.

7. Phosphate géminé insaturé de formule (III) suivant la revendication 1, dans lequel R₃ représente un groupe alkyle en C₁ à C₄.

8. Phosphate géminé insaturé de formule (III) suivant la revendication 1, qui est
l'ester tétraéthylique d'acide [5-benzoyl-2,4-dihydro-3H-pyrazole-3-ylidène]bisphosphonique, l'ester tétraéthylique d'acide [5-(cyclohexylcarbonyl)-2,4-dihydro-3H-pyrazole-3-ylidène]bisphosphonique,
l'ester tétraéthylique d'acide [5-[2-(2-fluoro-[1,1'-biphényl]-4-yl)-1-oxopropyl]-2,4-dihydro-3H-pyrazole-3-ylidène]bisphosphonique,
l'ester tétraéthylique d'acide [3-méthyl-5(4H)-isoxazolylidène]bisphosphonique,
l'ester tétraéthylique d'acide [3-phényl-5(4H)-isoxazolylidène]bisphonique,
l'ester tétraéthylique d'acide [2,4-dihydro-5-(1-oxopropyl)-3H-pyrazole-3-ylidène]bisphosphonique,
l'ester tétraéthylique d'acide [2,4-dihydro-5-(4-nitrobenzoyl)-3H-pyrazole-3-ylidène]bisphonique,
l'ester tétraéthylique d'acide [3-benzoyl-5(4H)-isoxazolylidène]bisphosphonique,
l'ester tétraéthylique d'acide [5-(4'-chlorobenzoyl)-2,4-dihydro-3H-pyrazole-3-ylidène]bisphosphonique,
l'ester tétraéthylique d'acide [5-(2',4'-dichlorobenzoyl)-2,4-dihydro-3H-pyrazole-3-ylidène]bisphosphonique,
l'ester tétraéthylique d'acide [5-(2,2-diméthyl-1-oxopropyl)-2,4-dihydro-3H-pyrazole-3-ylidène]bisphosphonique,
l'ester tétraéthylique d'acide [5-(2'-fluorobenzoyl)-2,4-dihydro-3H-pyrazole-3-ylidène]bisphosphonique,
l'ester tétraéthylique d'acide [5-(4'-méthoxybenzoyl)-2,4-dihydro-3H-pyrazole-3-ylidène]bisphosphonique,
l'ester tétraéthylique d'acide [5-(4'-méthylbenzoyl)-2,4-dihydro-3H-pyrazole-3-ylidène]bisphosphonique,
l'ester tétraéthylique d'acide [5-(3'-méthylbenzoyl)-2,4-dihydro-3H-pyrazole-3-ylidène]bisphosphonique,
l'ester tétraéthylique d'acide [5-(3'-fluorobenzoyl)-2,4-dihydro-3H-pyrazole-3-ylidène]bisphosphonique,
l'ester tétraéthylique d'acide [5-(2'-méthylbenzoyl)-2,4-dihydro-3H-pyrazole-3-ylidène]bisphosphonique,
l'ester tétraéthylique d'acide [5'-(4'-bromobenzoyl)-2,4-dihydro-3H-pyrazole-3-ylidène]bisphosphonique,
l'ester tétraéthylique d'acide [5'-(2',3',4'-trichlorobenzoyl)-2,4-dihydro-3H-pyrazole-3-ylidène]bisphosphonique,
l'ester tétraéthylique d'acide [5'-(3'-méthoxybenzoyl)-2,4-dihydro-3H-pyrazole-3-ylidène]bisphosphonique,
l'ester tétraéthylique d'acide [5'-(2',4'-diméthoxybenzoyl)-2,4-dihydro-3H-pyrazole-3-ylidène]bisphosphonique,
l'ester tétraéthylique d'acide [5'-(3'-trifluorométhylbenzoyl)-2,4-dihydro-3H-pyrazole-3-ylidène]bisphosphonique,
l'ester tétraéthylique d'acide [5'-(cyclopropanoyl)-2,4-dihydro-3H-pyrazole-3-ylidène]bisphosphonique,
l'ester tétraéthylique d'acide [5'-(9'-anthracénoyl)-2,4-dihydro-3H-pyrazole-3-ylidène]bisphosphonique,
l'ester tétraéthylique d'acide [5'-(3',5'-difluorobenzoyl)-2,4-dihydro-3H-pyrazole-3-ylidène]bisphosphonique.

9. Phosphate géminé insaturé de formule (III) suivant la revendication 1, qui est
l'ester tétraéthylique d'acide [5-benzoyl-2,4-dihydro-3H-pyrazole-3-ylidène]bisphosphonique,
l'ester tétraéthylique d'acide [5-(cyclohexylcarbonyl)-2,4-dihydro-3H-pyrazole-3-ylidène]bisphosphonique,
l'ester tétraéthylique d'acide [5-[2-(2-fluoro-[1,1'-biphényl]-4-yl)-1-oxopropyl]-2,4-dihydro-3H-pyrazole-3-ylidène]bisphosphonique,
l'ester tétraéthylique d'acide [3-méthyl-5(4H)-isoxazolylidène]bisphosphonique,
l'ester tétraéthylique d'acide [3-phényl-5(4H)-isoxazolylidène]bisphosphonique,
l'ester tétraéthylique d'acide [2,4-dihydro-5-(4-nitrobenzoyl)-3H-pyrazole-3-ylidène]bisphosphonique,
l'ester tétraéthylique d'acide [3-benzoyl-5(4H)-isoxazolylidène]bisphosphonique,
l'ester tétraéthylique d'acide [5-(4'-chlorobenzoyl)-2,4-dihydro-3H-pyrazole-3-ylidène]bisphosphonique,
l'ester tétraéthylique d'acide [5-(2',4'-dichlorobenzoyl)-2,4-dihydro-3H-pyrazole-3-ylidène]bisphosphonique,
l'ester tétraéthylique d'acide [5-(2',2'-diméthyl-1-oxopropyl)-2,4-dihydro-3H-pyrazole-3-ylidène]bisphosphonique,
l'ester tétraéthylique d'acide [5-(2'-fluorobenzoyl)-2,4-dihydro-3H-pyrazole-3-ylidène]bisphosphonique,
l'ester tétraéthylique d'acide [5-(4'-méthoxybenzoyl)-2,4-dihydro-3H-pyrazole-3-ylidène]bisphosphonique,
l'ester tétraéthylique d'acide [5-(4'-méthylbenzoyl)-2,4-dihydro-3H-pyrazole-3-ylidène]bisphosphonique,
l'ester tétraéthylique d'acide [5-(3'-méthylbenzoyl)-2,4-dihydro-3H-pyrazole-3-ylidène]bisphosphonique,
l'ester tétraéthylique d'acide [5-(3'-fluorobenzoyl)-2,4-dihydro-3H-pyrazole-3-ylidène]bisphosphonique,
l'ester tétraéthylique d'acide [5-(2'-méthylbenzoyl)-2,4-dihydro-3H-pyrazole-3-ylidène]bisphosphonique,
l'ester tétraéthylique d'acide [5'-(4'-bromobenzoyl)-2,4-dihydro-3H-pyrazole-3-ylidène]bisphosphonique,
l'ester tétraéthylique d'acide [5'-(2',3',4'-trichlorobenzoyl)-2,4-dihydro-3H-pyrazole-3-ylidène]bisphosphonique,
l'ester tétraéthylique d'acide [5'-(3'-méthoxybenzoyl)-2,4-dihydro-3H-pyrazole-3-ylidène]bisphosphonique,
l'ester tétraéthylique d'acide [5'-(2',4'-diméthoxybenzoyl)-2,4-dihydro-3H-pyrazole-3-ylidène]bisphosphonique,
l'ester tétraéthylique d'acide [5'-(3'-trifluorométhylbenzoyl)-2,4-dihydro-3H-pyrazole-3-ylidène]bisphosphonique,
l'ester tétraéthylique d'acide [5'-(cyclo-propanoyl)-2,4-dihydro-3H-pyrazole-3-ylidène]bisphosphonique,
l'ester tétraéthylique d'acide [5'-(9'-anthracénoyl)-2,4-dihydro-3H-pyrazole-3-ylidène]bisphosphonique,
l'ester tétraéthylique d'acide [5'-(3',5'-difluorobenzoyl)-2,4-dihydro-3H-pyrazole-3-ylidène]bisphosphonique.

10. Phosphonate géminé insaturé de formule (III) suivant la revendication 1, qui est
l'ester tétraéthylique d'acide [5-(2'-bromobenzoyl)-2,4-dihydro-3H-pyrazole-3-ylidène]bisphosphonique,
l'ester tétraéthylique d'acide [5-(3'-bromobenzoyl)-2,4-dihydro-3H-pyrazole-3-ylidène]bisphosphonique,
l'ester tétraéthylique d'acide [5-(2'-chlorobenzoyl)-2,4-dihydro-3H-pyrazole-3-ylidène]bisphosphonique,
l'ester tétraéthylique d'acdie [5-(3'-chlorobenzoyl)-2,4-dihydro-3H-pyrazole-3-ylidène]bisphosphonique,
l'ester tétraéthylique d'acide [5-(3',4'-diclorobenzoyl)-2,4-dihydro-3H-pyrazole-3-ylidène]bisphosphonique,
l'ester tétraéthylique d'acide [5-(3',5'-dichlorobenzoyl)-2,4-dihydro-3H-pyrazole-3-ylidène]bisphosphonique,
l'ester tétraéthylique d'acide [5-(2',6'-dichlorobenzoyl)-2,4-dihydro-3H-pyrazole-3-ylidène]bisphosphonique,
l'ester tétraéthylique d'acide (5-(4'-fluorobenzoyl)-2,4-dihydro-3H-pyrazole-3-ylidène]bisphosphonique,
l'ester tétraéthylique d'acide [5-(2',3',4',5',6'-pentafluorobenzoyl)-2,4-dihydro-3H-pyrazole-3-ylidène]bisphosphonique,
l'ester tétraéthylique d'acide [5-(2'-methoxybenzoyl)-2,4-dihydro-3H-pyrazole-3-ylidène]bisphosphonique,
l'ester tétraéthylique d'acide [5-(3',5'-diméthoxybenzoyl)-2,4-dihydro-3H-pyrazole-3-ylidène]bisphosphonique,
l'ester tétraéthylique d'acide [5-(2'-chloro-4'-méthoxybenzoyl)-2,4-dihydro-3H-pyrazole-3-ylidène]bisphosphonique,
l'ester tétraéthylique d'acide [5-(4'-chloro-2'-méthoxybenzoyl)-2,4-dihydro-3H-pyrazole-3-ylidène]bisphosphonique,
l'ester tétraéthylique d'acide [5-(3'-chloro-4'-méthoxybenzoyl)-2,4-dihydro-3H-pyrazole-3-ylidène]bisphosphonique,
l'ester tétraéthylique d'acide [5-(4'-chloro-3'-méthoxybenzoyl)-2,4-dihydro-3H-pyrazole-3-ylidène]bisphosphonique,
l'ester tétraéthylique d'acide [5-(3'-méthoxybenzoyl)-2,4-dihydro-3H-pyrazole-3-ylidène]bisphosphonique,
l'ester tétraéthylique d'acide [5-(2'-éthoxybenzoyl)-2,4-dihydro-3H-pyrazole-3-ylidène]bisphosphonique,
l'ester tétraéthylique d'acide [5-(3'-éthoxybenzoyl)-2,4-dihydro-3H-pyrazole-3-ylidène]bisphosphonique,
l'ester tétraéthylique d'acide [5-(4'-éthoxybenzoyl)-2,4-dihydro-3H-pyrazole-3-ylidène]bisphosphonique,
l'ester tétraéthylique d'acide [5-(2'-phénoxybenzoyl)-2,4-dihydro-3H-pyrazole-3-ylidène]bisphosphonique,
l'ester tétraéthylique d'acide [5-(3'-phénoxybenzoyl)-2,4-dihydro-3H-pyrazole-3-ylidène]bisphosphonique,
l'ester tétraéthylique d'acide [5-(4'-phénoxybenzoyl)-2,4-dihydro-3H-pyrazole-3-ylidène]bisphosphonique,
l'ester tétraéthylique d'acide [5-(2'-méthylthiobenzoyl)-2,4-dihydro-3H-pyrazole-3-ylidène]bisphosphonique,
l'ester tétraéthylique d'acide [5-(3'-méthylthiobenzoyl)-2,4-dihydro-3H-pyrazole-3-ylidène]bisphosphonique,
l'ester téraéthylique d'acide [5-(4'-méthylthiobenzoyl)-2,4-dihydro-3H-pyrazole-3-ylidène]bisphosphonique,
l'ester tétraéthylique d'acide [5-(2'-chloro-4'-méthylbenzoyl)-2,4-dihydro-3H-pyrazole-3-ylidène]bisphosphonique,
l'ester tétraéthylique d'acide [5-(4'-chloro-2'-méthylbenzoyl)-2,4-dihydro-3H-pyrazole-3-ylidène]bisphosphonique,
l'ester tétraéthylique d'acide [5-(3'-chloro-4'-méthylbenzoyl)-2,4-dihydro-3H-pyrazole-3-ylidène]bisphosphonique,
l'ester tétraéthylique d'acide [5-(4'-chloro-3'-méthylbenzoyl)-2,4-dihydro-3H-pyrazole-3-ylidène]bisphosphonique,
l'ester tétraéthylique d'acide [5-(2'-éthylbenzoyl)-2,4-dihydro-3H-pyrazole-3-ylidène]bisphosphonique,
l'ester tétraéthylique d'acide [5-(3'-éthylbenzoyl)-2,4-dihydro-3H-pyrazole-3-ylidène]bisphosphonique,
l'ester tétraéthylique d'acide [5-(4'-éthylbenzoyl)-2,4-dihydro-3H-pyrazole-3-ylidène]bisphosphonique,
l'ester tétraéthylique d'acide [5-(4'-tertiobutylbenzoyl)-2,4-dihydro-3H-pyrazole-3-ylidène]bisphosphonique,
l'ester tétraéthylique d'acide [5-(2',4'-diméthylbenzoyl)-2,4-dihydro-3H-pyrazole-3-ylidène]bisphosphonique,
l'ester tétraéthylique d'acide [5-(3',5'-diméthylbenzoyl)-2,4-dihydro-3H-pyrazole-3-ylidène]bisphosphonique,
l'ester tétraéthylique d'acide [5-(2'-phénylbenzoyl)-2,4-dihydro-3H-pyrazole-3-ylidène]bisphosphonique,
l'ester tétraéthylique d'acide [5-(3'-phénylbenzoyl)-2,4-dihydro-3H-pyrazole-3-ylidène]bisphosphonique,
l'ester tétraéthylique d'acide [5-(4'-phénylbenzoyl)-2,4-dihydro-3H-pyrazole-3-ylidène]bisphosphonique,
l'ester tétraéthylique d'acide [5-(4'-morpholinobenzoyl)-2,4-dihydro-3H-pyrazole-3-ylidène]bisphosphonique,
l'ester tétraéthylique d'acide [5-(4'-diméthylaminobenzoyl)-2,4-dihydro-3H-pyrazole-3-ylidène]bisphosphonique,
l'ester tétraéthylique d'acide [5-(4'-diéthylaminobenzoyl)-2,4-dihydro-3H-pyrazole-3-ylidène]bisphosphonique,
l'ester tétraéthylique d'acide [5-(1'-naphtoyl)-2,4-dihydro-3H-pyrazole-3-ylidène]bisphosphonique,
l'ester tétraéthylique d'acide [5-(2'-naphtoyl)-2,4-dihydro-3H-pyrazole-3-ylidène]bisphosphonique,
l'ester tétraéthylique d'acide [5-(6'-quinolinoyl)-2,4-dihydro-3H-pyrazole-3-ylidène]bisphosphonique,
l'ester tétraéthylique d'acide [5-(8'-quinolinoyl)-2,4-dihydro-3H-pyrazole-3-ylidène]bisphosphonique,
l'ester tétraéthylique d'acide [5-(2'-thiénylcarbonyl)-2,4-dihydro-3H-pyrazole-3-ylidène]bisphosphonique,
l'ester tétraéthylique d'acide [5-(2'-pyridylcarbonyl)-2,4-dihydro-3H-pyrazole-3-ylidène]bisphosphonique,
l'ester tétraéthylique d'acide [5-(nicotinoyl)-2,4-dihydro-3H-pyrazole-3-ylidène]bisphosphonique,
l'ester tétraéthylique d'acide [5-(4'-pyridylcarbonyl)-2,4-dihydro-3H-pyrazole-3-ylidène]bisphosphonique,
l'ester tétraéthylique d'acide [5-(cyclobutanoyl)-2,4-dihydro-3H-pyrazole-3-ylidène]bisphosphonique, et
l'ester tétraéthylique d'acide [5-(cyclopentanoyl)-2,4-dihydro-3H-pyrazole-3-ylidène]bisphosphonique.

11. Ester tétraéthylique d'acide [5-(3'-fluorobenzoyl)-2,4-dihydro-3H-pyrazole-3-ylidène]bisphosphonique.

12. Ester tétraéthylique d'acide [5'-(2',3',4'-trichlorobenzoyl)-2,4-dihydro-3H-pyrazole-3-ylidène]bisphosphonique.
